# EUROPÄISCHE PATENTSCHRIFT

(11) **EP 2 240 179 B1**
(45) Veröffentlichungstag und Bekanntmachung des Hinweises auf die Patenterteilung: **17.08.2016**
(21) Anmeldenummer: 09707983.4
(22) Anmeldetag: 23.01.2009
(51) Int. Cl.: A61K 31/5025, A61P 7/02, C07D 487/04

(54) **IMIDAZOPYRIDAZINE ALS PAR1-INHIBITOREN, IHRE HERSTELLUNG UND VERWENDUNG ALS ARZNEIMITTEL**
IMIDAZOPYRIDAZINE AS PAR1 INHIBITORS, THEIR MANUFACTURE AND THEIR APPLICATION AS MEDICINE
IMIDAZOPYRIDAZINE EN TANT QU'INHIBITEURS DE PAR1, SON PROCÉDÉ DE FABRICATION ET SON UTILISATION EN TANT QUE MÉDICAMENT

(30) Priorität: 05.02.2008 EP 08290113
(43) Veröffentlichungstag der Anmeldung: 20.10.2010
(73) Patentinhaber: SANOFI, 75008 Paris (FR)
(72) Erfinder: HEINELT, Uwe, 65926 Frankfurt am Main (DE); WEHNER, Volkmar, 65926 Frankfurt am Main (DE); HERRMANN, Matthias, 65926 Frankfurt am Main (DE); SCHOENAFINGER, Karl, 63755 Alzenau (DE); STEINHAGEN, Henning, 65843 Sulzbach (DE)
(74) Vertreter: Then, Johann
(86) Internationale Anmeldenummer: PCT/EP2009/000409
(87) Internationale Veröffentlichungsnummer: WO 2009/097973

(56) Entgegenhaltungen:
- EP-A- 1 391 451
- EP-A- 1 394 152
- EP-A- 1 813 282
- CHACKALAMANNIL S ET AL: "Thrombin receptor (PAR-1) antagonists as novel antithrombotic agents" EXPERT OPINION ON THERAPEUTIC PATENTS, INFORMA HEALTHCARE, GB, Bd. 16, Nr. 4, 1. April 2006 (2006-04-01), Seiten 493-505, XP002415653 ISSN: 1354-3776

## Beschreibung

Die Erfindung betrifft neue Verbindungen der Formel I 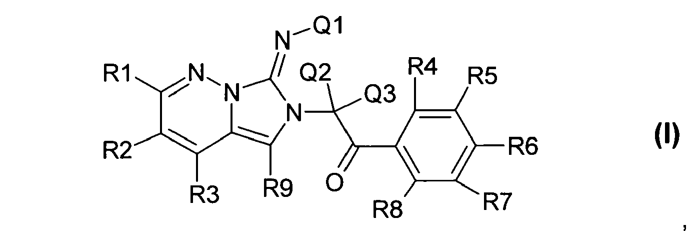 wobei R1, R2, R3, R4, R5, R6, R7, R8, R9, Q1, Q2 und Q3 die unten bezeichnete Bedeutung haben. Die Verbindungen der Formel I haben antithrombotische Aktivität und inhibieren insbesondere den Protease-aktivierten Rezeptor 1 (PAR1). Die Erfindung betrifft ferner ein Verfahren zur Herstellung der Verbindung der Formel I und die Verbindung der Formel I zur Verwendung als Arzneimittel.

Der Protease-aktivierte Rezeptor 1 (PAR1) ist ein Thrombin Rezeptor, der zur Klasse der G Protein-gekoppelten Rezeptoren (GPCR) gehört. Das Gen für PAR1 liegt auf Chromosom 5q13, besteht aus zwei Exonen und deckt eine Region von etwa 27 kb ab.

PAR1 wird unter anderem in Endothelzellen, glatten Muskelzellen, Fibroblasten, Neuronen und humanen Blutplättchen exprimiert. Auf Blutplättchen ist PAR1 ein wichtiger Rezeptor der Signalübertragung welcher an der Initiation der Aggregation von Blutplättchen beteiligt ist.

Die Aktivierung der PARs erfolgt über die proteolytische Abspaltung eines Teils des N-Terminus der PARs, wodurch eine neue N-terminale Sequenz freigelegt wird, die dann den Rezeptor aktiviert (Pharmacol Rev 54:203-217, 2002).

Die Blutgerinnung ist ein für das Überleben von Säugetieren wesentlicher Vorgang der Kontrolle des Blutstroms. Der Vorgang der Gerinnung und der nachfolgenden Auflösung des Gerinnsels nach erfolgter Wundheilung setzt nach einer Gefäßschädigung ein und lässt sich in vier Phasen einteilen:
1. Die Phase der vaskulären Konstriktion: Hierdurch wird der Blutverlust in das geschädigte Areal vermindert.
2. Die nächste Phase ist die der Plättchenadhäsion an das freigelegte Kollagen im Subendothel. Diese primäre Adhäsion an die Matrix aktiviert die Plättchen, die daraufhin verschiedene Aktivatoren sekretieren, die zur Verstärkung der Aktivierung führen. Diese Aktivatoren stimulieren zudem die weitere Rekrutierung neuer Plättchen zum Ort der Gefäßschädigung und fördern die Plättchenaggregation. Die Plättchen aggregieren an der Stelle des Gefäßwandschadens und bilden ein noch lockeres Plättchengerinnsel. Weiterhin führt die Aktivierung der Plättchen zur Präsentation von Phosphatidylserin und Phosphatidylinositol entlang der Zellmembranoberflächen. Die Exposition dieser Phospholipide ist zur Bindung und Aktiverung von Multienzymkomplexen der Blutgerinnungskaskade essentiell.
3. Das anfänglich noch lockere Plättchenaggregat wird durch Fibrin vernetzt. Wenn der Thrombus lediglich Plättchen und Fibrin enthält, handelt es sich um einen weißen Thrombus. Sind zusätzlich rote Blutkörperchen vorhanden, handelt es sich um einen roten Thrombus.
4. Nach Wundheilung wird der Thrombus durch die Einwirkung des Proteins Plasmin aufgelöst.

Zwei alternative Wege führen zur Bildung eines Fibringerinnsels, der intrinsische und der extrinsische Weg. Diese Wege werden durch unterschiedliche Mechanismen eingeleitet, in späterer Phase konvergieren sie jedoch zu einer gemeinsamen Wegstrecke der Gerinnungskaskade. Die Bildung eines roten Thrombus oder eines Gerinnsels auf dem Boden einer Gefäßwandabnormität ohne Wunde ist das Resultat des intrinsischen Weges. Die Fibringerinnselbildung als Antwort auf einen Gewebsschaden oder eine Verletzung ist das Resultat des extrinsischen Weges. Beide Wege beinhalten eine größere Anzahl von Proteinen, die als Gerinnungsfaktoren bekannt sind.

Der intrinsische Weg erfordert die Gerinnungsfaktoren VIII, IX, X, XI und XII sowie Präkallekrein, hochmolekulares Kininogen, Calciumionen und Phospholipide aus Plättchen. Jedes dieser Proteine führt zur Aktivierung des Faktors X.

Der intrinsische Weg wird eingeleitet, wenn Präkallekrein, hochmolekulares Kininogen Faktor XI und XII an eine negativ geladene Oberfläche binden. Dieser Moment wird als Kontaktphase bezeichnet. Die Exposition gegenüber einem Gefäßwandkollagen ist der primäre Stimulus der Kontaktphase. Resultat der Vorgänge der Kontaktphase ist die Umwandlung von Präkallekrein in Kallekrein, das wiederum den Faktor XII aktiviert. Faktor Xlla hydrolysiert weiteres Präkallekrein zu Kallekrein, so dass eine Aktivierung die Folge ist. Mit zunehmender Aktivierung von Faktor XII kommt es zur Aktivierung des Faktors XI, der zu einer Freisetzung von Bradykinin, einem Vasodilatator führt. Dadurch kommt es zur Beendigung der initialen Phase der Vasokonstriktion. Bradykinin entsteht aus dem hochmolekularen Kininogen. In Anwsenheit von Ca²⁺-Ionen aktiviert der Faktor Xla den Faktor IX. Faktor IX ist ein Proenzym, das Vitamin-K abhängige, c-Karboxiglutamat (GLA)-Reste enthält. Die Serinproteaseaktivität kommt nach Bindung von Ca²⁺-Ionen an diese GLA-Reste zum Tragen. Mehrere der Serinproteasen der Blutgerinnungskaskade (Faktoren II, VII, IX und X) enthalten derartige Vitamin-K-abhängige GLA-Reste. Faktor IXa spaltet den Faktor X und führt zur Aktivierung zum Faktor Xa. Voraussetzung für die Bildung von Faktor IXa ist die Bildung eines Proteasekomplexes aus von Ca²⁺-Ionen und den Faktoren VIIIa, IXa und X an der Oberfläche aktivierter Plättchen. Eine der Reaktionen aktivierter Plättchen ist die Präsentation von Phosphatidylserin und Phosphatidylinositol entlang der Oberflächen. Die Exposition dieser Phospholipide macht erst die Bildung des Proteasekomplexes möglich. Faktor VIII hat in diesem Vorgang die Funktion eines Rezeptors für die Faktoren IXa und X. Faktor VIII stellt daher einen Cofaktor in der Gerinnungskaskade dar. Die Aktivierung des Faktors VIII mit Bildung des Faktors VIIIa, dem eigentlichen Rezeptor, bedarf nur einer minimalen Menge von Thrombin. Mit Zunahme der Konzentration von Thrombin wird der Faktor VIIIa schließlich durch Thrombin weiter gespalten und inaktiviert. Diese duale Aktivität des Thrombins in Bezug zum Faktor VIII führt zu einer Selbstbegrenzung der Proteasekomplexbildung und damit zu einer Eingrenzung der Blutgerinnung.

Bei der Aktivierung von humanen Blutplättchen durch Thrombin spielen PAR1 und PAR4 eine zentrale Rolle; die Aktivierung dieser Rezeptoren führt in Blutplättchen zu morphologischen Veränderungen, Freisetzung von ADP und Aggregation der Blutplättchen (Nature 413:26-27, 2001).

Inhibitoren von PAR 1 werden beispielsweise in den Europäischen Patentanmeldungen EP1391451, EP1394152 oder EP 1 813 282, den amerikanischen Patentanmeldungen US 6,063,847 und US 2004/0152736 sowie der Internationalen Anmeldung WO 03/089428 und in Chackalamannil S. et al., Expert Opin. Ther. Patents, Informa. Healthcare, GB, 2006, Bd. 16(4), 493-505 beschrieben.

Die Verbindungen der Formel I zeigen eine hohe spezifische Inhibierung des Protease-aktivierten Rezeptor 1 und eignen sich daher für die prophylaktische als auch für die therapeutische Anwendung am Menschen, die an Erkrankungen leiden, die mit Thrombosen, Embolien, Hyperkoagulabilität oder fibrotischen Veränderungen einhergehen. Beispiele solcher Erkrankungen sind Thrombose, tiefe Venenthrombose, Lungenembolien, Gehirninfarkt, Herzinfarkt, Bluthochdruck, Entzündliche Erkrankungen, Rheuma, Asthma, Glomerulonephritis oder Osteoporose. Die Verbindungen der Formel I können zur Sekundär-Prävention eingesetzt werden und eignen sich sowohl für eine akute als auch für eine Langzeittherapie.
Die Verbindungen der Formel I sind auch in Kombination mit Wirkstoffen einsetzbar, die über andere antithrombotische Prinzipien wie PAR 1 wirken.

### 1) Die Erfindung betrifft daher eine Verbindung der Formel I

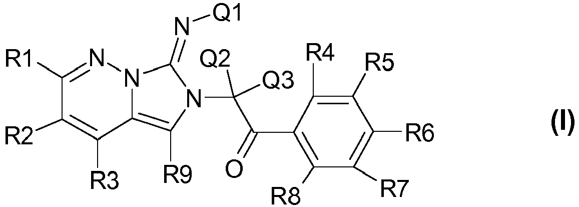

und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
Q1, Q2 und Q3 gleich oder verschieden sind und unabhängig voneinander für Wasserstoffatom oder -(C₁-C₆)-Alkyl stehen, wobei in Alkyl die Wasserstoffatome ganz oder teilweise durch Fluor ersetzt sein können,
R1, R2 und R3 gleich oder verschieden sind und unabhängig voneinander für Wasserstoffatom, -(C₁-C₆)-Alkyl, -(C₃-C₆)-Cycloalkyl, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-Alkylen-C(O)-R11, -(C₀-C₄)-Alkylen-N(R11 )-R12, -(C₀-C₄)-Alkylen-N(R11 )-C(O)-R12, Halogen, OH, -CN, -(C₁-C₆)-Alkylen-O-(C₁-C₆)-Alkyl, -O-(C₁-C₆)-Alkylen-O-(C₁-C₆)-Alkyl oder -O-(C₁-C₄)-Alkylen-(C₃-C₆)-Cycloalkyl,
   stehen,
   wobei Alkyl, Alkylen und Cycloalkyl jeweils unsubstituiert oder ein-, zwei- oder dreifach unabhängig voneinander durch -(C₁-C₄)-Alkyl, OH oder -O-(C₁-C₆)-Alkyl substituiert sind, oder
   wobei in Alkyl, Alkylen oder Cycloalkyl die Wasserstoffatome ganz oder teilweise durch Fluor ersetzt sein können,
   mit der Maßgabe, das mindestens ein R1, R2 oder R3 nicht Wasserstoffatom ist
R11 und R12 unabhängig voneinander für Wasserstoffatom, -(C₁-C₆)-Alkyl oder -(C₃-C₆)-Cycloalkyl stehen,
R4, R5, R6, R7 und R8 gleich oder verschieden sind und unabhängig voneinander für Wasserstoffatom, -(C₁-C₆)-Alkyl, -(C₃-C₆)-Cycloalkyl, OH, -CN, -O-(C₁-C₈)-Alkyl, -O-(C₃-C₆)-Cycloalkyl, -(C₀-C₄)-Alkylen-(CO)-N(R21)-R22, -(C₀-C₄)-Alkylen-C(O)-O-R21, Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -(C₀-C₆)-Alkylen-O-(C₁-C₆)-Alkylen-O-(C₁-C₆)-Alkyl, oder -(C₀-C₆)-Alkylen-O-(C₁-C₄)-Alkylen-(C₃-C₆)-Cycloalkyl stehen,
   wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, oder
   wobei in Alkyl, Alkylen oder Cycloalkyl die Wasserstoffatome ganz oder teilweise durch Fluor ersetzt sein können,
   mit der Maßgabe, das mindestens ein R4, R5, R6, R7 oder R8 nicht Wasserstoffatom ist,
R9 für Wasserstoffatom oder -(C₁-C₆)-Alkyl steht, wobei in Alkyl die Wasserstoffatome ganz oder teilweise durch Fluor ersetzt sein können,
R21 und R22 unabhängig voneinander für ein Wasserstoffatom, -(C₁-C₆)-Alkyl oder -(C₃-C₆)-Cycloalkyl stehen,
   wobei in Alkyl oder Cycloalkyl die Wasserstoffatome ganz oder teilweise durch Fluor ersetzt sein können, oder
R21 und R22 in den Fragmenten "N(R21)-R22" und "N(R21)-C(O)-R22" für einen Ring stehen, ausgewählt aus der Gruppe Pyrrolidinyl, Piperidinyl, Piperazinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können.

Die Erfindung betrifft ferner eine Verbindung der Formel I, wobei
Q1, Q2 und Q3 gleich sind und für jeweils ein Wasserstoffatom stehen,
R1, R2 und R3 gleich oder verschieden sind und unabhängig voneinander für Wasserstoffatom, -(C₁-C₄)-Alkyl, -O-CH₂-CF₃ oder -O-(C₁-C₆)-Alkyl stehen,
mit der Maßgabe, das mindestens ein R1, R2 oder R3 nicht Wasserstoffatom ist,
R4 und R8 jeweils für ein Wasserstoff steht, R5, R6 und R7 gleich oder verschieden sind und unabhängig voneinander für Wasserstoffatom, -(C₁-C₆)-Alkyl, -O-(C₁-C₄)-Alkyl, -SF₅ oder -N(R21)-R22 stehen,
mit der Maßgabe, das mindestens ein R5, R6 oder R7 nicht Wasserstoffatom ist, und R9 für Wasserstoffatom steht,
R21 und R22 unabhängig voneinander für Wasserstoffatom oder -(C₁-C₄)-Alkyl stehen, oder
R21 und R22 in dem Fragment "N(R21 )-R22" für einen Ring stehen, ausgewählt aus der Gruppe Pyrrolidinyl, Piperidinyl, Piperazinyl, Imidazolyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl.

Außerordentlich bevorzugt sind Verbindungen der Formel I, wobei folgende Verbindungen umfasst sind,
1-(3-tert-Butyl-4-methoxy-5-morpholin-4-yl-phenyl)-2-(7-imino-2,3-dimethoxy-imidazo[1,5-b]pyridazin-6-yl)-ethanon als Trifluoressigsäuresalz,
1-(3-tert-Butyl-4-methoxy-5-morpholin-4-yl-phenyl)-2-(2,3-diethoxy-7-imino-imidazo[1,5-b]pyridazin-6-yl)-ethanon als Trifluoressigsäuresalz,
N-{3-[2-(2,3-Diethoxy-7-imino-imidazo[1,5-b]pyridazin-6-yl)-acetyl]-5-pentafluorsulfanyl-phenyl}-acetamid als Trifluoressigsäuresalz,
1-(3-tert-Butyl-4-methoxy-5-morpholin-4-yl-phenyl)-2-[7-imino-2-methoxy-3-(2,2,2-trifluoro-ethoxy)-imidazo[1,5-b]pyridazin-6-yl]-ethanon, 2-(2,3-Diethoxy-7-imino-imidazo[1,5-b]pyridazin-6-yl)-1-(5-methylamino-3-pentafluorsulfanyl-phenyl)-ethanon oder
2-(2,3-Diethoxy-7-imino-imidazo[1,5-b]pyridazin-6-yl)-1-[3-methylamino-5-(pentafluoro-sulfanyl)-phenyl]-ethanon.

Unter dem Begriff "(C₁-C₄)-Alkyl" oder "(C₁-C₆)-Alkyl" werden Kohlenwasserstoffreste verstanden, deren Kohlenstoffkette geradkettig oder verzweigt ist und 1 bis 4 Kohlenstoffatome oder 1 bis 6 Kohlenstoffatome enthält, beispielsweise Methyl, Ethyl, Propyl, Isopropyl, Butyl, Isobutyl, sec-Butyl, tertiär-Butyl, Pentyl, Isopentyl, Neopentyl, 1-Ethylpropyl, Hexyl, 2,3-Dimethylbutyl oder Neohexyl.
Unter dem Begriff "-(C₀-C₄)-Alkylen" oder "-(C₁-C₆)-Alkylen" werden Kohlenwasserstoffreste verstanden, deren Kohlenstoffkette geradkettig oder verzweigt ist und 1 bis 4 oder 1-6 Kohlenstoffatome enthält, beispielsweise Methylen, Ethylen, 1-Methylmethylen, Propylen, 1-Methylethylen, Butylen, 1-Propylmethylen, 1-Ethyl-1-methylmethylen, 1,2-Dimethylethylen, 1,1-Dimethylmethylen, 1-Ethylethylen, 1-Methylpropylen, 2-Methylpropylen, Pentylen, 1-Methylbutylen, Hexylen, 1-Methylpentylen. "-C₀-Alkylen" ist eine kovalente Bindung.
Unter dem Begriff "-O-(C₁-C₆)-Alkyl" oder "-O-(C₁-C₈)-Alkyl" werden Alkoxyreste verstanden, deren Kohlenstoffkette geradkettig oder verzweigt ist und 1 bis 6 oder 1 bis 8 Kohlenstoffatome enthält, beispielsweise Methoxy, Ethoxy, Propoxy, Iso-Propoxy, Butoxy, Iso-Butoxy, tertiär-Butoxy, 1-Pentoxy, 2-Pentoxy, 3-Pentoxy, 1-Hexoxy, 2-Hexoxy, 3-Hexoxy, 1-Heptoxy, 2-Heptoxy, 3-Heptoxy, 4-Heptoxy, 2,4-Dimethyl-pentan-3-oxy, 1-Octoxy, 2-Octoxy, 3-Octoxy, 2,2,4-Trimethyl-pentan-3-oxy, 2,3,4-Trimethyl-pentan-3-oxy oder 4-Octoxy.
Unter dem Begriff "(C₃-C₆)-Cycloalkyl" werden Reste verstanden wie Verbindungen, die sich von 3- bis 6-gliedrige Monocyclen wie Cyclopropan, Cyclobutan, Cyclopentan oder Cyclohexan herleiten.
Unter dem Begriff "-O-(C₃-C₆)-Cycloalkyl" werden Cycloalkoxy-Reste verstanden wie Verbindungen, die sich von 3- bis 6-gliedrige Monocyclen wie Cyclopropoxy, Cyclobutoxy, Cyclopentoxy oder Cyclohexoxy herleiten.
"R21 und R22 in den Fragmenten "N(R21)-R22" und "N(R21)-C(O)-R22" stehen für einen Ring, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten", wobei die cyclische Amine ausgewählt sind aus Pyrrolidinyl, Piperidinyl, Piperazinyl, Morpholinyl oder Thiomorpholinyl, wobei die Imide ausgewählt sind aus Pyrrolidin-2,5-dionyl, Piperidin-2,6-dionyl, Piperazin-2,6-dionyl, Morpholin-3,5-dionyl, und wobei die Lactame ausgewählt sind aus Pyrrolidin-2-onyl, Piperidin-2-onyl, Piperazin-2-onyl, Morpholin-3-onyl.
Unter der Umschreibung "Alkyl, Alkylen oder Cycloalkyl die Wasserstoffatome ganz oder teilweise durch Fluor ersetzt sein" wird ein partiell oder vollständig fluorierter Alkyl-, Alkylen- oder Cycloalkylrest verstanden, der sich beispielsweise für Alkyl von folgenden Resten -CF₃, -CHF₂, -CH₂F, -CHF-CF₃, -CHF-CHF₂, -CHF-CH₂F, -CH₂-CF₃, -CH₂-CHF₂, -CH₂-CH₂F, -CF₂-CF₃, -CF₂-CHF₂, -CF₂-CH₂F, -CH₂-CHF-CF₃, -CH₂-CHF-CHF₂, -CH₂-CHF-CH₂F, -CH₂-CH₂-CF₃, -CH₂-CH₂-CHF₂, -CH₂-CH₂-CH₂F, -CH₂-CF₂-CF₃, -CH₂-CF₂-CHF₂, -CH₂-CF₂-CH₂F, -CHF-CHF-CF₃, -CHF-CHF-CHF₂, -CHF-CHF-CH₂F, -CHF-CH₂-CF₃, -CHF-CH₂-CHF₂, -CHF-CH₂-CH₂F, -CHF-CF₂-CF₃, -CHF-CF₂-CHF₂, -CHF-CF₂-CH₂F, -CF₂-CHF-CF₃, -CF₂-CHF-CHF₂, -CF₂-CHF-CH₂F, -CF₂-CH₂-CF₃, -CF₂-CH₂-CHF₂, -CF₂-CH₂-CH₂F, -CF₂-CF₂-CF₃, -CF₂-CF₂-CHF₂, -CF₂-CF₂-CH₂F, -CH(CF₃)₂, -CH(CHF₂)₂, -CH(CFH₂)₂, -CH(CFH₂)(CHF₂), -CH(CFH₂)(CF₃), -CH(CFH₂)(CH₃), -CH(CHF₂)(CH₃), -CH(CF₃)(CH₃), -CF(CF₃)₂, -CF(CHF₂)₂, -CF(CFH₂)₂, -CF(CFH₂)(CHF₂), -CF(CFH₂)(CF₃), -CF(CFH₂)(CH₃), -CF(CHF₂)(CH₃), -CF(CF₃)(CH₃), sowie die weiteren möglichen Kombinationen für Butyl, Pentyl und Hexyl, die wie Propyl auch verzweigt sein können,
für Alkylen beispielsweise von folgenden Resten -CF₂-, -CHF-, -CHF-CF₂-, -CHF-CHF-, -CHF-CH₂-, -CF₂-CF₂-, -CF₂-CH₂F, sowie die weiteren möglichen Kombinationen für Propylen, Butylen, Pentylen und Hexylen, die auch verzweigt sein können, und für Cycloalkyl beispielsweise von den Resten 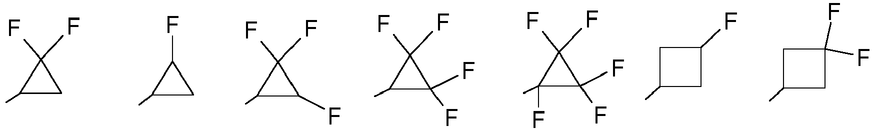 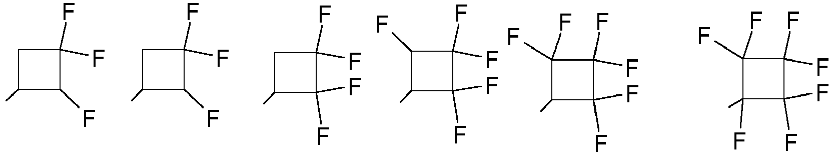 sowie den analogen größeren Ringen Cyclopentyl und Cyclohexyl ableitet.

Unter dem Begriff "Hal" wird Fluor, Chlor, Brom oder Jod verstanden, bevorzugt sind Fluor, Chlor oder Brom, insbesondere Fluor oder Chlor.

Oben beschriebene Begriffe sind auch beliebig kombinierbar wie beispielsweise in "-(C₀-C₆)-Alkylen-O-(C₀-C₆)-Alkylen-(C₆-C₁₄)-Aryl".

Funktionelle Gruppen der verwendeten Intermediate, beispielsweise Amino- oder Carboxylgruppen in der Verbindung der Formel I, können dabei durch geeignete Schutzgruppen maskiert werden. Geeignete Schutzgruppen für Aminofunktionen sind beispielweise die t-Butoxycarbonyl-, die Benzyloxycarbonyl- oder die Phtalolylgruppe sowie die Trityl- oder Tosylschutzgruppe. Geeignete Schutzgruppen für die Carboxylfunktion sind beispielweise Alkyl-, Aryl- oder Arylalkylester. Schutzgruppen können durch wohlbekannte oder hier beschriebene Techniken eingeführt und entfernt werden (siehe Greene, T.W., Wuts, P.G.M., Protective Groups in Organic Synthesis (1999), 3rd Ed., Wiley-Interscience, oder Kocienski, P. J., Protecting Groups (2004), 3rd Ed., Thieme. Der Begriff Schutzgruppe kann auch entsprechende polymergebundene Schutzgruppen umfassen.
Die erfindungsgemäßen Verbindungen können nach wohlbekannten Verfahren oder nach hier beschriebenen Verfahren hergestellt werden.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man
a) eine Verbindung der Formel II 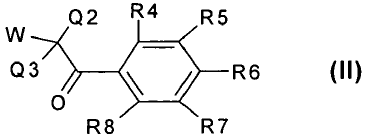 wobei R4, R5, R6, R7, R8, Q2 und Q3 wie in Formel I definiert sind und W für Chlorid, Bromid, Mesylat oder Tosylat steht, mit einer Verbindung der Formel III, 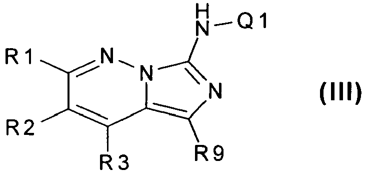 wobei R1, R2, R3, R9 und Q1 wie in Formel I definiert sind, mit oder ohne Basenzugabe in einem Lösungsmittel zu einer Verbindung der Formel I umsetzt, oder
b) eine Verbindung der Formel VII, 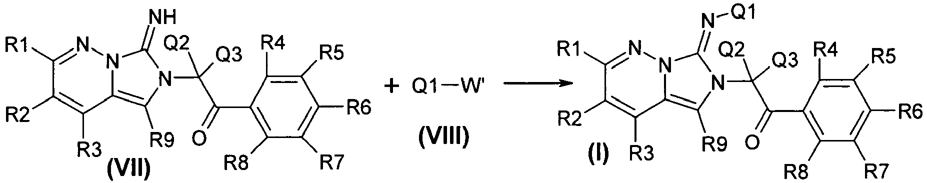 wobei R1, R2, R3, R4, R5, R6, R7, R8, R9, Q2 und Q3 wie in Formel I definiert sind, mit einer Verbindung Q1-W', wobei W' Chlorid, Bromid, Tosylat, Mesylat, Methylsulfat oder eine ähnliche gute Fluchtgruppe bedeutet, mit oder ohne Basenzugabe zu einer Verbindung der Formel I umsetzt, oder
c) eine Verbindung der Formel XXVI, 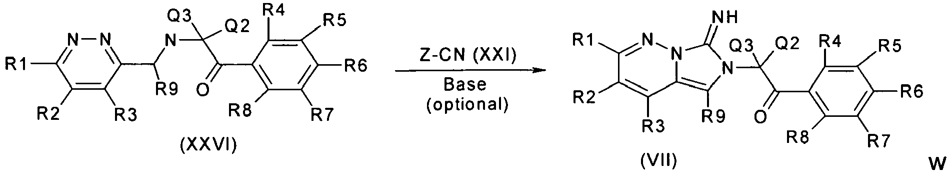 obei R1, R2, R3, R4, R5, R6, R7, R8, R9, Q2 und Q3 wie in Formel I definiert sind, mit einer Verbindung Z-CN, wobei Z eine gute Fluchtgruppe wie Tosylat, Chlorid oder Bromid bedeutet, mit oder ohne Basenzugabe zu einer Verbindung der Formel VII umsetzt, oder
d) eine Verbindung der Formel XXVII, 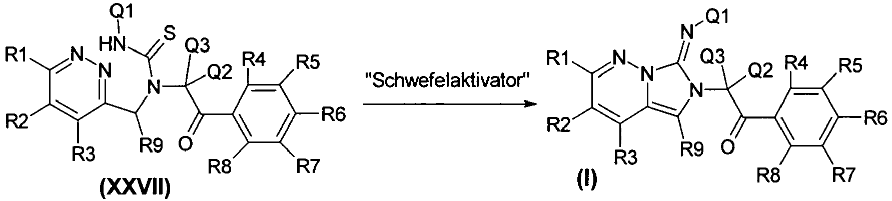 wobei R1, R2, R3, R4, R5, R6, R7, R8, R9, Q1, Q2 und Q3 wie in Formel I definiert sind, mit einem Schwefelaktivator zu einer Verbindung der Formel I umsetzt, oder
e) die nach den Verfahren a) bis d) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
f) eine nach den Verfahren a) bis d) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

Die Erfindung betrifft ferner ein Verfahren zur Herstellung der Verbindung der Formel I nach Schema 1.

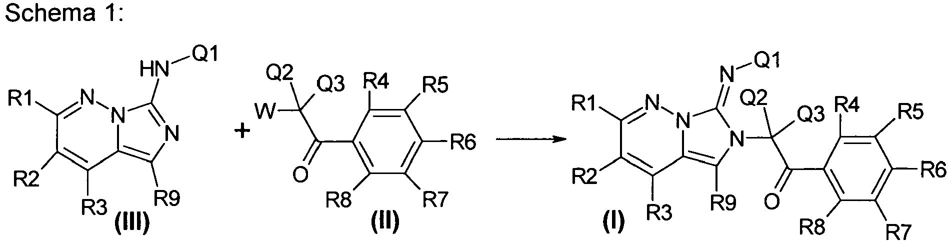

Die Edukte II und III, wobei II gegebenenfalls (ggf.) in Form eines Salzes vorliegt, werden dabei bei Raumtemperatur oder leicht erhöhter Temperatur von 40 °C bis 60 °C vorteilhafterweise, wenn II als Salz vorliegt, in Gegenwart einer Base, bevorzugt Hünig-Base, in einem Lösungsmittel, bevorzugt Dimethylformamid (DMF), THF oder Dioxan, zu der Verbindung der Formel I umgesetzt. Die Reste R1, R2, R3, R4, R5, R6, R7, R8, R9, Q1, Q2 und Q3 sind wie in Formel I definiert, W entspricht einer guten Fluchtgruppe wie Chlorid, Bromid, Mesylat oder Tosylat, bevorzugt Bromid oder Mesylat.

Verbindungen der Formel II sind käuflich oder lassen sich nach literaturbekannten Verfahren beispielsweise ausgehend von den korrespondierenden Acetophenonen X oder X' gewinnen (siehe beispielsweise: Phosphorus and Sulfur and the Related Elements (1985), 25(3), 357 oder Tetrahedron Letters (1984), 25(34), 3715). Die wohlbekannten und in zahlreichen Strukturvariationen käuflichen Verbindungen vom Typ X lassen sich beispielsweise an der Acetylgruppe unter anderem mit elementarem Chlor, Brom, Tribromid-Derivaten wie Phenyl-trimethylammonium-tribromid, 1,3-Dichlordimethylhydantoin, N-Chlor- oder N-Bromsuccinimid funktionalisieren. Verbindungen von Typ X' lassen sich beispielsweise mit Mesyl- oder Tosylchlorid in die Verbindungen vom Typ II überführen.

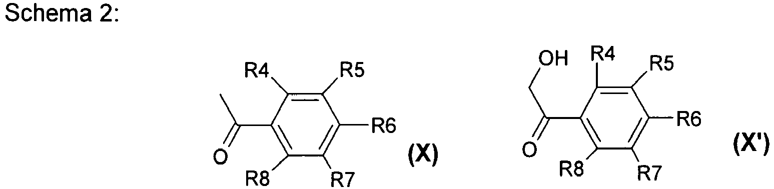

Für bestimmte Reste R4 bis R8 kann es günstiger sein, die Ketone vom Typ X erst in die Ketale vom Typ XI oder XI' zu überführen, die dann sehr selktiv an der Methylgruppe zu den Verbindungen vom Typ XII funktionalisiert werden können, bevorzugt bromiert, und nach Deketalisierung mit geeigneten Säuren ebenfalls zu Verbindungen vom Typ II führen.
Die Substituenten in Schemata 2 und 3 sind dabei wie oben definiert, T entspricht einer -(C₁-C₄)-Alkyl-Gruppe, während T' für Ethylen, Propylen oder Butylen steht, W' entspricht einer reaktiven Verbindung wie Phenyl-trimethylammonium-tribromid, N-Brom- oder N-Chlorsuccinimid.

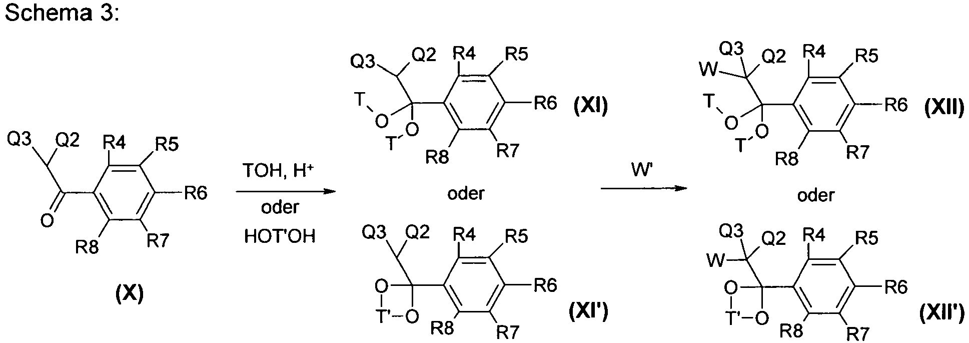

Zur Synthese von Verbindungen vom Typ der Formel III' (Typ Formel III mit Q1 = H) werden Verbindungen der Formel XX - ggf. in Form ihrer Salze (HA) - bevorzugt in Gegenwart einer Base mit einer Cyan-Quelle vom Typ XXI zu den gewünschten Imidazopyridinen cyclisiert. Als Säuren HA kommen bevorzugt HBr, HCl, Trifluoressigsäure (TFA) und Schwefelsäure in Frage. Z steht für eine gute Fluchtgruppe, bevorzugt Tosylat, Chlorid oder Bromid.

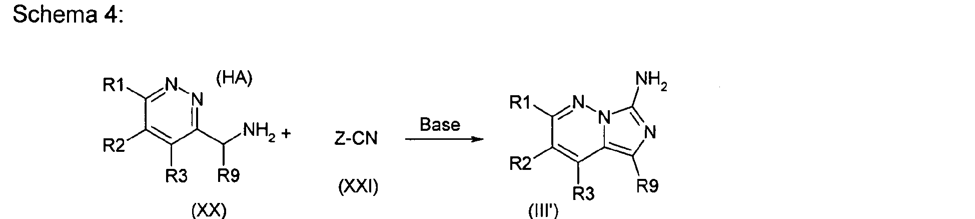

Alternativ lassen sich Verbindungen vom Typ der Formel III durch Umsetzungen mit Isothiocyanaten vom Typ der Formel XXII gewinnen, wobei intermediär Thioharnstoffe vom Typ XXIII gebildet werden. Diese lassen sich dann mit "Schwefelaktivatoren" wie Methyliodid, Quecksilberoxid oder Bromessigsäureethylester in die gewünschten Verbindungen vom Typ der Formel III überführen.

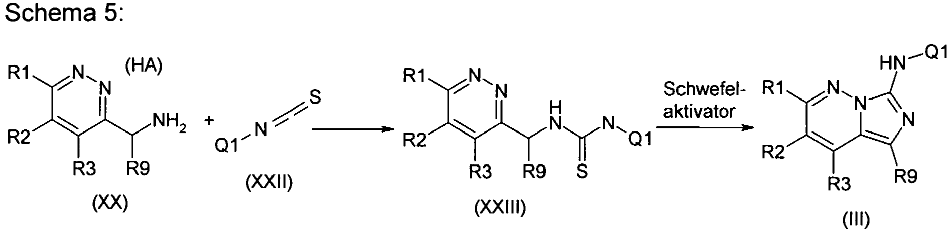

Verbindungen vom Typ der Formel XX sind käuflich oder können entsprechend Schema 6 gewonnen werden. 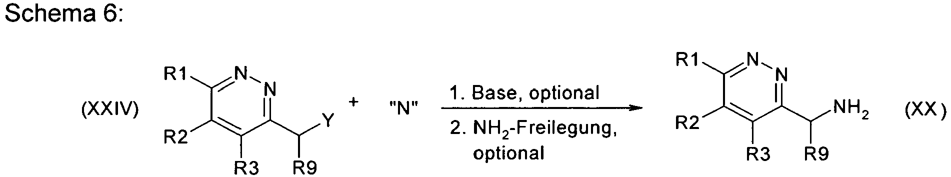 Dabei werden Pyridazine vom Typ XXIV in Gegenwart eines Stickstoff-Nucleophils "N" und ggf. einer Folgereaktion zur Freilegung der -NH₂-Gruppe in die Pyridazinylmethylamine XX übergeführt. Als Stickstoff-Nukleophile kommen dabei Ammonik, das direkt ohne weitere Freilegung zu den Verbindungen vom Typ XX führt, Azide, wie Natriumazid, das zur Etablierung der Aminofunktion nachträglich reduziert werden muss, wozu Triphenylphosphin (Bioorg. Med. Chem. Lett. 2925, 2002) oder Edelmetallkatalysatoren wie Palladium oder Platin in Gegenwart von Wasserstoff in Frage kommen (J. Med. Chem. 5005, 2002), Phthalimid, das nachträglich zur Freilegung der Aminofunktion mit Hydrazin behandelt werden muss (J. Med. Chem. 1315, 2004), oder Urotropin, das zur Freilegung der Aminofunktion mit Säure, bevorzugt Salzsäure, behandelt werden muss (Synthesis 2145, 2003), in Frage. Die Reste R1, R2, R3 und R9 sind wie in Formel I definiert, und Y steht für eine gute Fluchtgruppe wie Chlorid, Bromid, Mesylat oder Tosylat und kann hier auch -OH entsprechen, das "in situ" zu einer guten Fluchtgruppe aktiviert wird, die dann nachfolgend von einem der oben erwähnten Stickstoff-Nucleophile substituiert wird (Chem. Pharm. Bul 1493, 1989; Bioorg. Med. Chem. Lett. 2463, 2004).
Ein weiterer Zugang zu Aminen von Typ XX ist in Schema 7 dargestellt.

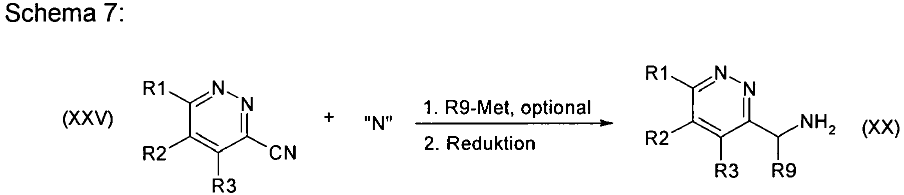

Ausgehend von 3-Cyanopyridazinen vom Typ XXV wird die Nitrilfunktion mit Reduktionsmitteln wie Wasserstoff in Gegenwart von Metallkatalysatoren wie Palladium oder Raney-Nickel zu den Aminen vom Typ XX reduziert. Wird die Nitrilfunktion vor der Reduktion mit metallorganischen Reagenzien wie Grignard- oder Organolithium-Verbindungen umgesetzt, lässt sich auch auf diesem Weg der Substituent R9 einführen. Die dadurch intermediär erhaltenen Imine lassen sich durch Natriumborhydrid, Natriumtriacetoxyborhydrid oder Natriumcyanoborhydrid zu den Aminen XX reduzieren. Die Reste R1, R2, R3 und R9 sind wie oben definiert. Met steht für -Li oder -MgBr.

Alternativ lassen sich Verbindungen der Formel I wie in Schema 8 dargestellt herstellen.

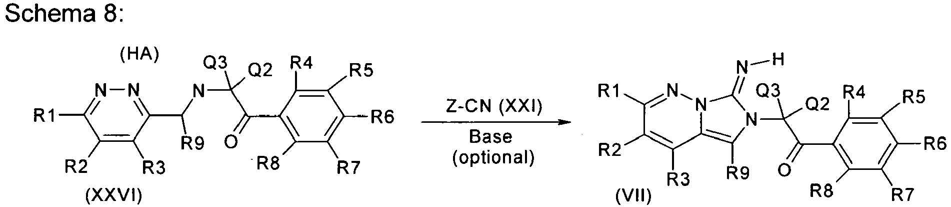

Dabei werden Verbindungen der Formel XXVI - ggf. in Form ihrer Salze (HA)- in einem Lösungsmittel wie Wasser, Methanol, Ethanol, Essigsäure, Acetonitril, Toluol oder geeigneten Gemischen dieser Lösungsmittel, bevorzugt Toluol, in Gegenwart einer Base, bevorzugt Hünig-Base, mit einer Cyan-Quelle XXI, bevorzugt Bromcyan zu den gewünschten Imidazopyridazinen cyclisiert. Die Reste R1 bis R9, Q2, Q3 und Z sind wie oben definiert.

Verbindungen der Formel XXVI werden entsprechend Schema 9 erhalten, indem Amine der Formel XX mit Acetophenon-Derivaten vom Typ der Formel II umgesetzt werden. Dies geschieht bevorzugt in Lösungsmitteln wie DMF, Tetrahydrofuran (THF) oder Acetonitril, bevorzugt in THF. Als Basen kommen Hünig-Base, Lithiumhexamethyldisilazan oder Kaliumcarbonat in Frage, bevorzugt Lithiumhexamethyldisilazan. Die Reste sind dabei wie oben definiert.

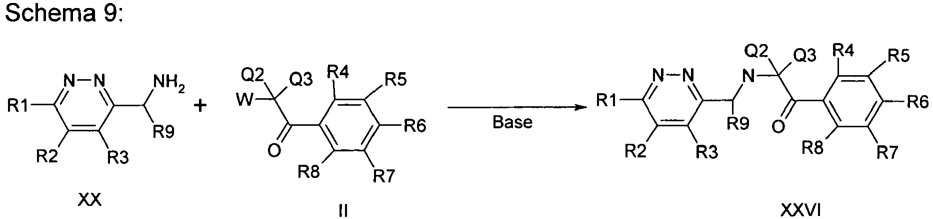

Alternativ lassen sich Verbindungen der Formel XXVI laut Schema 10 mit Isothiocyanaten vom Typ der Formel XXII zu den Thioharnstoffen XXVII umsetzen. Diese werden nachfolgend mit einem "Schwefelaktivator" wie Quecksilberoxid, Methyliodid oder Bromessigsäureethylester behandelt, so dass sie direkt zu Verbindungen vom Typ der Formel I cyclisieren. Die Reste sind dabei wie oben definiert.

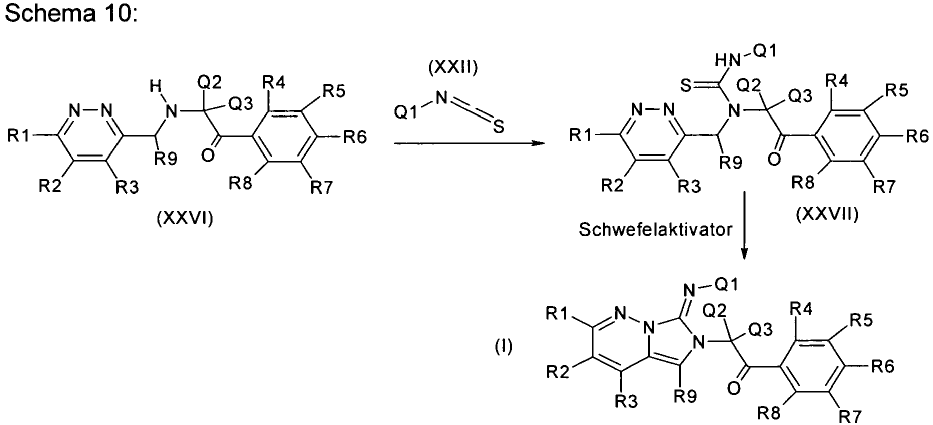

Die Verbindungen der Formel I können teilweise auch in isomeren Formen auftreten, wobei Q1 in der folgenden Teilformel von Formel I entweder (E) oder (Z) konfiguriert sein kann:

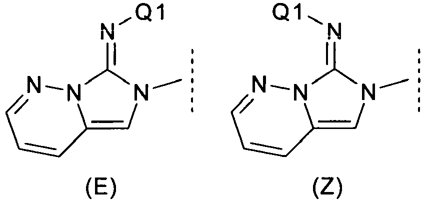

Eine nach Schema 1 hergestellte Verbindung der Formel I, oder eine geeignete Vorstufe der Formel I, die aufgrund ihrer chemischen Struktur in enantiomeren Formen auftritt, kann durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren und Abspaltung der chiralen Hilfsgruppen in die reinen Enantiomeren aufgetrennt werden (Verfahren f)), oder die nach Schema 1 hergestellte Verbindung der Formel I kann entweder in freier Form isoliert oder im Falle des Vorliegens von sauren oder basischen Gruppen in physiologisch verträgliche Salze umwandelt werden (Verfahren e)).

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze oder Hydrochloride, Sulfate, Hemisulfate, Methylsulfonate, p-Toluolsulfonate, alle möglichen Phosphate sowie Salze der Aminosäuren, natürlicher Basen oder Carbonsäuren wie Lactate, Citrate, Tartrate, Acetate, Adipinate, Fumarate, Gluconate, Glutamate, Maleinate oder Pamoate.
Die Herstellung physiologisch verträglicher Salze aus zur Salzbildung befähigten Verbindungen der Formel I, einschließlich deren stereoisomeren Formen, gemäß Verfahrensschritt e) erfolgt in an sich bekannter Weise. Enthalten Verbindungen der Formel I saure Funktionalität, so lassen sich mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze bilden. Basische Gruppen der Verbindungen der Formel I, bilden mit Säuren Säureadditionssalze. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon- 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron-, Palmitin- oder Trifluoressigsäure in Frage.

Im Verfahrensschritt f) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, kann auch eine fraktionierte Kristallisation der mit einer optisch aktiven Base oder Säure als Hilfsstoff gebildeten diastereomeren Salze durchgeführt werden. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Amin-Funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.
Weiterhin ergibt sich bei einigen der erfindungsgemäßen Verbindungen die Möglichkeit, zur Herstellung der Gerüststrukturen diastereo- oder enantiomerenreine Ausgangsprodukte einzusetzen. Dadurch können auch andere oder vereinfachte Verfahren zur Aufreinigung der Endprodukte eingesetzt werden. Diese Ausgangsprodukte wurden zuvor nach literatur-bekannten Verfahren enantiomeren- oder diastereomerenrein hergestellt. Das kann insbesondere bedeuten, dass in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen beispielsweise zur Prophylaxe, Sekundär-Prävention und Therapie all solcher Erkrankungen, die durch eine Hemmung des Protease-aktivierten Rezeptors 1 (PAR1) behandelbar sind. So eignen sich die erfindungsgemäßen Verbindungen sowohl für eine prophylaktische als auch für eine therapeutische Anwendung am Menschen. Sie eignen sich sowohl für eine akute Behandlung als auch für eine Langzeittherapie. Die Verbindungen der Formel I können eingesetzt werden in Patienten, die an Störungen des Wohlbefindens oder Krankheiten leiden, die mit Thrombosen, Embolien, Hyperkoagulabilität, fibrotischen Veränderungen oder entzündlichen Erkrankungen einhergehen.
Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen. Weiterhin können die Verbindungen der Formel I eingesetzt werden bei allen Eingriffen, die zu einem Kontakt des Blutes mit Fremdoberflächen führen wie bei Dialysepatienten und Patienten mit Verweilkathetern. Verbindungen der Formel I können eingesetzt werden, um die Thrombosegefahr nach chirurgischen Eingriffen wie bei Knie- und Hüftgelenksoperationen zu reduzieren.
Verbindungen der Formel I eignen sich für die Behandlung von Patienten mit disseminierter intravaskulärer Koagulation, Sepsis und anderen intravaskulären Ereignissen, die mit einer Entzündung einhergehen. Weiterhin eignen sich Verbindungen der Formel I für die Prophylaxe und Behandlung von Patienten mit Atherosklerose, Diabetes und dem metabolischen Syndrom und deren Folgen. Störungen des hämostatischen Systems (beispielsweise Fibrinablagerungen) wurden impliziert in Mechanismen, die zu Tumorwachstum und Tumormetas-tasierung führen, sowie bei entzündlichen und degenerativen Gelenkserkrankungen wie der rheumatoiden Arthritis und der Arthrose. Verbindungen der Formel I eignen sich zur Verlangsamung oder Verhinderung solcher Prozesse.
Weitere Indikationen für den Einsatz der Verbindungen der Formel I sind fibrotische Veränderungen der Lunge wie die chronische obstruktive Lungenerkrankung, das adult respiratory distress syndrome (ARDS) und des Auges wie Fibrinablagerungen nach Augenoperationen. Verbindungen der Formel I eignen sich auch zur Verhinderung und/oder Behandlung von Narbenbildung.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation. Eine Beschichtung von Stents mit Verbindungen der Formel I und anderen Oberflächen, die im Körper mit Blut in Kontakt kommen, ist möglich.
Die Erfindung betrifft auch ein Verfahren zur Herstellung eines Arzneimittels, das dadurch gekennzeichnet ist, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.
Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.
Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.
Für die Behandlung eines erwachsenen, etwa 70 kg schweren Patienten sind je nach Wirksamkeit der Verbindung gemäß Formel I, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Verbindungen der Formel I können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden. Als Plättchenaggregationshemmer kommen dabei Cyclooxygenase 1-Inhibitoren wie Aspirin, irreversible P2Y₁₂-Antagonisten wie Clopidogrel oder Prasugrel, reversible P2Y₁₂-Antagonisten wie Cangrelor oder AZD6140 und Thromboxan A₂/Prostaglandin H₂-Antagonisten wie Terutroban in Frage. Additive Effekte von PAR 1-Blockade in Kombination mit P2Y₁₂-Blockade konnten beispielweise bereits gezeigt werden (Eur. Heart J. 2007, 28, Abstract Supplement, 188).

### Beispiele

Endprodukte wurden in der Regel durch eine chromatographisch-massenspektroskopische Methode (LCUV/ESI-MS-Kopplung) und ¹H-NMR charakterisiert. Beschrieben sind die Verbindungen durch Angabe der zugehörigen Retentionszeit im Ionenstrom (LCMS-Rt) und des entsprechenden M+H⁺-Signals bei positiver lonisiation im zugehörigen Massenspektrum. Konnte kein M+H⁺-Massensignal erhalten werden, wurden alternativ die ¹H-NMR-Daten angegeben. Verwendete Abkürzungen sind entweder erläutert oder entsprechen den üblichen Konventionen. Kieselgeltrennungen wurden manuell (Flash-Chromatographie) oder unterstützt durch halbautomatische Kartuschen-Systeme wie Companion (CombiFlash) oder Flashmaster II (Jones Chromatography) durchgeführt. Soweit nicht anders angegeben, wurden chromatographische Trennungen an Kieselgel mit Ethylacetat/Heptan-, Dichlormethan/Ethanol- oder Dichlormethan/Methanol-Gemischen als Laufmittel durchgeführt.
Das Abdampfen von Lösungsmitteln geschah in der Regel unter vermindertem Druck bei 35 °C bis 45 °C am Rotationsverdampfer und wird mit "vom Lösungsmittel befreit", "eingeengt", "einrotiert", "zur Trockne gebracht", "Lösungsmittel entfernt bzw. abgezogen" oder ähnlichen Ausdrücken umschrieben. Wenn nicht anders aufgeführt, wurden die LCUV/MS-Analysen unter folgenden Bedingungen durchgeführt:

| | |
|---|---|
| System: | Agilent 1100 HPLC-System gekoppelt an 1100 LC/MSD |
| Säule: | YMC J'shere ODS H80 20x2,1 mm, Packungsmaterial 4 µm |
| Laufmittel: | ACN:H₂O+0,05% TFA (Fluss 1 ml/min) |
| Gradient: | 4:96 (0 min) → 95:5 (2 min) → 95:5 (2,4 min) →4:96 (2,45 min) |
| Ionisierung: | ESI⁺ |

Präparative HPLC mit Reversed-Phase-(RP)-Kieselgel wurde mit den folgenden Methoden durchgeführt:

**Methode A, Standard-Methode, wenn im Text keine andere erwähnt ist.**

| | |
|---|---|
| Säule: | Merck (Darmstadt, Deutschland) Purosphere® RP18 25x250 mm, 10 µm |
| Laufmittel: | ACN:H₂O+0,05%TFA (Fluss 25 ml/min) |
| Gradient: | 10:90 (0 min) → 90:10 (40 min) |

**Methode B**

| | |
|---|---|
| Säule: | Merck Purosphere® RP18 25x250 mm, 10 µm |
| Laufmittel: | ACN:H₂O+0,05%TFA (Fluss 25 ml/min) |
| Gradient: | 0:100 (0 min) → 0:100 (5 min) → 20:80 (20 min) |

**Methode C**

| | |
|---|---|
| Säule: | Agilent Prep-C18, 30 x 250mm, 10 µM |
| Laufmittel: | ACN:H₂O+0,05%TFA (Fluss 75 ml/min) |
| Gradient: | 10:90 (0 min) → 90:10 (12,5 min) → 90:10 (15min) → 10:90 (15,5 min) → 10:90 (17.5 min) |

Die Umsetzungen fanden in Standard-Reaktionsapparaturen wie Ein- oder Mehrhalskolben statt, die wenn nicht anders beschrieben, dem Bedarf angemessen 5 ml bis 2000 ml Volumen fassten und je nach Anforderung mit Septum, Stopfen, Kühler, Rührer oder anderen Ausrüstungsgegenständen bestückt waren. Wenn nicht anders erwähnt, fanden alle Reaktionen unter Argon als Schutzgas statt und wurden mit Magnetrührern gerührt.

Mikrowellenreaktionen wurden im Emrys Optimizer von Personal Chemistry in dem Bedarf angepassten Gefäßen von 0,5 bis 10 ml Fassungsvermögen durchgeführt.

### Verwendete Abkürzungen:

- abs.: absolut
- ACN: Acetonitril
- Boc: Butoxycarbonyl
- DCM: Dichlormethan
- DIPEA: N,N-Diisopropylethylamin (Hünig-Base)
- DMF: Dimethylformamid
- DMSO: Dimethylsulfoxid
- EE: Essigsäureethylester
- HPLC: Hochleistungsflüssigkeitschromatographie
- LCMS-Rt: Retentionszeit der Verbindung im Ionenstrom
- LCUV/MS: Flüssigkeitschromatographie Ultraviolett-/Massenspektroskopie
- MeOH: Methanol
- RT: Raumtemperatur (20 °C bis 25 °C)
- TFA: Trifluoressigsäure
- THF: Tetrahydrofuran

### Beispiel 1

### N-{3-[2-(2,3-Diethoxy-7-imino-imidazo[1,5-b]pyridazin-6-yl)-acetyl]-5-pentamethyl-sulfanyl-phenyl}-acetamid als Trifluoressigsäuresalz

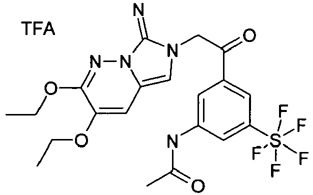

### a) 3-Nitro-5-pentafluorsulfanyl-benzoesäure

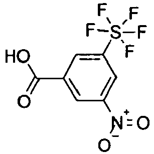

3-Pentafluorsulfanyl-benzoesäure (5,0 g) wurde in rauchender Salpetersäure (20 ml) gelöst und bei RT unter Feuchtigkeitsausschluss gerührt. Dann wurde konzentrierte Schwefelsäure (3 ml) zugegeben und bei 75 °C gerührt. Nach 5 h Rühren bei 75°C wurde weitere Schwefelsäure (1,5 ml) zugegeben und nach 2 h Rühren bei 75°C über Nacht stehen gelassen. Dann wurde auf Eiswasser gegeben und 2 h gerührt. Der gebildete Niederschlag wurde abgesaugt und am Hochvakuum getrocknet. Es wurden 4,2 g 3-Pentafluorsulfanyl-5-nitro-benzoesäure erhalten. Weitere 900 mg konnten aus der Mutterlauge nach dreimaligem Extrahieren mit Methylenchlorid, Trockenen der vereinigten Methylenchlorid-Phasen über Magnesiumsulfat und Einengen des Lösungsmittels erhalten werden. Der Niederschlag wurde ohne weitere Reinigung in der nächsten Stufe eingesetzt.
¹H-NMR (400 MHz, DMSO-d₆) [ppm]: 8,82 (1 H); 8,80 (1 H); 8,62 (1 H)

### b) N-Methoxy-N-methyl-5-nitro-3-pentafluorsulfanyl-benzamid

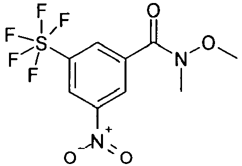

3-Nitro-5-pentafluorsulfanyl-benzoesäure (4,0 g) wurde unter Rühren in Thionyl-chlorid (25ml) gelöst und 10 h unter Feuchtigkeitsausschluss unter Rückfluss gehalten. Nach Stehen über Nacht bei RT wurde überschüssiges Thionylchlorid im Vakuum entfernt, der erhaltene Rückstand in Dichlormethan (50 ml) gelöst und unter Rühren mit N,O-Dimethylhydroxylamin-Hydrochlorid (1,25 g) und Diethylisopropyl-amin (1,66 g) versetzt. Nach 1 h Rühren bei RT wurde das Gemisch im Vakuum eingeengt, der Rückstand in Essigester gelöst und 5 Mal mit Wasser gewaschen. Die organische Phase wurde über Magnesiumsulfat getrocknet, filtriert und eingeengt. Das erhaltene Rohprodukt (4,2 g) wurde direkt in der nächsten Stufe eingesetzt. LCMS-Rt: 1,50 min [M+H]⁺: 337,0

### c) 3-Amino-N-methoxy-N-methyl-5-pentafluorsulfanyl-benzamid

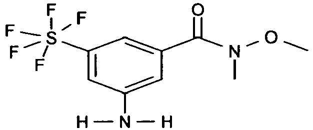

N-Methoxy-N-methyl-5-nitro-3-pentafluorsulfanyl-benzamid (4,2 g) wurde in Methanol (120 ml) gelöst und Raney - Nickel (etwa 700 mg) zugegeben. Mit aufgesetztem Wasserstoffballon wurde auf einem Magnetrührer hydriert. Nach 5 h wurde der Katalysator abfiltriert und mit Methanol gewaschen. Das Filtrat wurde im Vakuum eingeengt und der Rückstand mittels präparativer HPLC aufgereinigt. Die produktenthaltenden Fraktionen wurden vereinigt, vom Acetonitril befreit, mit Natriumhydrogencarbonat-Lösung basisch gestellt und mit Essigester drei Mal extrahiert. Die vereinigten Extrakte wurden über Magnesiumsulfat getrocknet, filtriert und eingeengt. Es wurden 1,73 g der gewünschten Verbindung erhalten.
LCMS-Rt: 1,27 min [M+H]⁺: 307,0

### d) 3-Acetylamino-N-methoxy-N-methyl-5-(pentafluorsulfanyl)-benzamid

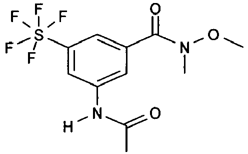

3-Amino-N-methoxy-N-methyl-5-pentafluorsulfanyl-benzamid (1,2 g) wurde in Methylenchlorid (15 ml) gelöst und unter Rühren Triethylamin (0,7 ml) gefolgt von Essigsäureanhydrid (1,75 ml) unter Feuchtigkeitsausschluss zugesetzt. Nach 3 h Rühren bei RT wurden Wasser und gesättigte Natriumhydrogencarbonat-Lösung zugegeben, die Phasen getrennt und die Methylenchlorid-Phase noch drei Mal mit Wasser gewaschen, über Magnesiumsulfat getrocknet, filtriert und eingeengt. Das erhaltene Produkt (1,3 g) wurde ohne weitere Reinigung in der nächsten Stufe eingesetzt. LCMS-Rt: 1,26 min [M+H]⁺: 349,0

### e) N-[3-Acetyl-5-(pentafluorsulfanyl)-phenyl]-acetamid

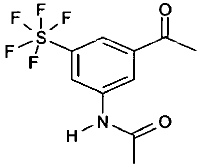

3-Acetylamino-N-methoxy-N-methyl-5-(pentafluorsulfanyl)-benzamid (1,2 g) wurde in absolutem THF (30 ml) gelöst und bei 0 °C mit Lithiumhexmethyldisilazan (721 µl; Dichte: 0,8 g/l; 23%-ig in *tert*.-Butylmethylether) 30 min gerührt. Bei 0°C wurde dann unter Rühren Methylmagnesiumbromid (2,87 ml, 3 M in Diethylether) zugetropft. Nach 2,5 h Rühren bei RT wurde weiteres Methylmagnesiumbromid (1 ml, 3 M in Diethylether) zugegeben und erneut 2,5 h gerührt. Zur Aufarbeitung wurde unter Eiskühlung 1 N Salzsäure zugetropft, gefolgt von Wasser und Essigester. Die organische Phase wurde abgetrennt und die Wasserphase noch zwei Mal mit Essigester extrahiert. Die vereinigten Essigesterphasen wurden über Natriumsulfat getrocknet, filtriert und eingeengt. Das Rohprodukt (1,03 g) wurde mit einem in gleicher Weise hergestellten Rohprodukt (75 mg) vereinigt und über Kieselgel mit Dichlormethan-Methanol als Laufmittel aufgereinigt. Es wurden 860 mg der gewünschten Verbindung erhalten. LCMS-Rt: 1,34 min [M+H]⁺: 304,0

### f) N-[3-(2-Brom-acetyl)-5-(pentafluorsulfanyl)-phenyl]-acetamid

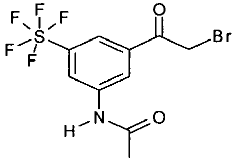

N-[3-Acetyl-5-(pentafluorsulfanyl)-phenyl]-acetamid (859 mg) wurde in einem Gemisch aus Methanol (10 ml) und THF (10 ml) gelöst und unter Rühren portionsweise Phenyltrimethyl-ammonium-tribromid (1,065 g) zugegeben. Nach 2 h Rühren bei RT wurde weitere 3 h auf 40°C erwärmt. Nach dem Erkalten wurde das Reaktionsgemisch in 2 N Schwefelsäure gegeben und die wässrige Phase 3 Mal mit Essigester extrahiert. Die vereinigten Extrakte wurden über Natriumsulfat getrocknet, filtriert und eingeengt. Das Rohprodukt wurde über Kieselgel mit Essigester/Heptan als Laufmittel aufgereinigt. Es wurden 480 mg der gewünschten Verbindung erhalten. LCMS-Rt: 1,47 min [M+H]⁺: 382,0

### g) 3,4-Diethoxy-6-methyl-pyridazin-1-oxid

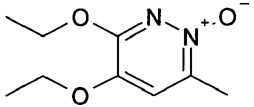

3-Methoxy-6-methyl-4-nitropyridazin-1-oxid (2 g) wurde in Ethanol (100 ml) bei RT vorgelegt. Danach wurde unter Rühren portionsweise festes Natriumethylat (1,4 g) zugegeben. Nach 1,5 h Rühren bei 55°C wurde das Reaktionsgemisch auf RT abgekühlt, mit Wasser versetzt und die wässrige Phase 3 Mal mit Dichlormethan extrahiert. Die vereinigten organischen Phasen wurden über Natriumsulfat getrocknet, filtriert und eingeengt. Das Rohprodukt wurde mittels präparativer HPLC aufgereinigt. Die sauberen produktenthaltenden Fraktionen wurden vereinigt, vom Acetonitril befreit und drei Mal mit Dichlormethan extrahiert. Die vereinigten organischen Phasen wurden über Natriumsulfat getrocknet, filtriert und eingeengt. Es wurden 176 mg des gewünschten Produkts erhalten. Die wässrige Phase wurde gefriergetrocknet und lieferte 781 mg des gewünschten Produkts.
Zur Gewinnung weiteren Produkts wurden die verunreinigten Fraktionen aus der Chromatographie vereinigt und gefriergetrocknet. Der Rückstande wurde erneut mittels präparativer HPLC aufgereinigt und das saubere Produkt wie oben beschrieben isoliert. Es wurden weitere 130 mg Produkt erhalten.
LCMS-Rt: 0,72 min [M+H]⁺: 199,1

### h) (5,6-Diethoxy-pyridazin-3-yl)-methanol

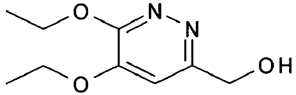

3,4-Diethoxy-6-methyl-pyridazin-1-oxid (1,44 g) wurde in Dichlormethan (50 ml) vorgelegt und bei RT tropfenweise mit Trifluoressigsäureanhydrid (2,5 ml) versetzt. Nach 2,5 h wurde zur Trockne gebracht und der Rückstand mit Ethanol und gesättigter Kaliumcarbonat-Lösung aufgenommen. Nach 4 h Rühren bei RT wurde erneut zur Trockne gebracht und der Rückstand mit Wasser und Dichlormethan versetzt. Nach Abtrennen der organischen Phase wurde noch zweimal mit Dichlormethan extrahiert, die vereinigten organischen Phasen wurden über Magnesiumsulfat getrocknet, filtriert und eingeengt. Der Rückstand wurde zweimal mit Dichlormethan/Methanol als Laufmittel über Kieselgel gereinigt. Die produktenthaltenden Fraktionen wurden vereinigt und zur Trockne gebracht. Es wurden 716 mg des gewünschten Produkts erhalten. LCMS-Rt: 0,57 min [M+H]⁺: 199,1

### i) Methansulfonsäure 5,6-diethoxy-pyridazin-3-ylmethyl-ester

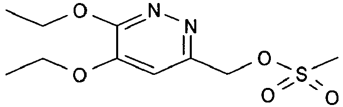

(5,6-Diethoxy-pyridazin-3-yl)-methanol (716 mg) wurde in Dichlormethan (35 ml) vorgelegt und nacheinander unter Rühren Methansulfonsäureanhydrid (1,7 g in 5 ml Dichlormethan gelöst) und Triethylamin (0,8 ml) zugetropft. Nach 2 h wurde mit Wasser und gesättigter Natriumhydrogencarbonat-Lösung versetzt und drei Mal mit Dichlormethan extrahiert. Die vereinigten organischen Phasen wurden über Natriumsulfat getrocknet, filtriert und eingeengt. Es wurden 1,1 g der gewünschten Verbindung erhalten. LCMS-Rt: 0,98 min [M+H]⁺: 277,0

### j) C-(5,6-Diethoxy-pyridazin-3-yl)-methylamin

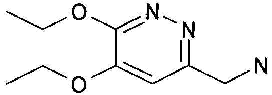

Methansulfonsäure 5,6-diethoxy-pyridazin-3-ylmethyl-ester (620 mg) wurde in Methanol (10 ml) gelöst und unter Eiskühlung zu einer Ammoniak-Lösung (16 ml; 7 N in Methanol) getropft. Nach 6 h Rühren und Stehenlassen über Nacht wurde das Lösungsmittel abgezogen und der Rückstand mittels präparativer HPLC aufgereinigt. Die produktenthaltenden Fraktionen wurden vereinigt, vom Acetonitril befreit und gefriergetrocknet. Es wurde 305 mg des gewünschten Produkts erhalten.
LCMS-Rt: 0,62 min [M+H]⁺: 198,1

### k) 2,3-Diethoxy-imidazo[1,5-b]pyridazin-7-ylamin als Trifluoressigsäuresalz

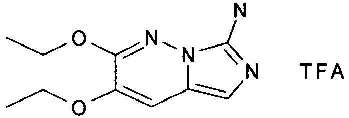

C-(5,6-Diethoxy-pyridazin-3-yl)-methylamin (296 mg) wurde in einer Mischung aus Ethanol (15 ml) und Wasser (3 ml) unter Rühren bei RT vorgelegt. Danach tropfte man Bromcyan (0,8 ml; 5 M in Acetonitril) zu. Nach 6 h Rühren und Stehenlassen über Nacht wurde weiteres Bromcyan (0,8 ml) zugegeben. Nach 9 h Rühren und Stehen über Nacht wurde das Lösungsmittel abgezogen und der Rückstand mittels präparativer HPLC aufgereinigt. Die produktenthaltenden Fraktionen wurden vereinigt, vom Acetonitril befreit und gefriergetrocknet. Das noch verunreinigte Produkt wurde zur weiteren Aufreinigung über Kieselgel mit Dichlormethan-Methanol als Laufmittel getrennt. Es wurden 99 mg der gewünschten Verbindung erhalten. LCMS-Rt: 0,83 min [M+H]⁺: 223,1

### I) N-{3-[2-(2,3-Diethoxy-7-imino-imidazo[1,5-b]pyridazin-6-yl)-acetyl]-5-pentamethylsulfanyl-phenyl}-acetamid als Trifluoressigsäuresalz

2,3-Diethoxy-imidazo[1,5-b]pyridazin-7-ylamin-Trifluoressigsäuresalz (22 mg) wurde in absolutem DMF (2,5 ml) bei RT unter Rühren vorgelegt und mit Diisopropylethyl-amin (5 µl) versetzt. Danach tropfte man N-[3-(2-Brom-acetyl)-5-(pentafluorsulfanyl)-phenyl]-acetamid (25 mg), gelöst in absolutem DMF (1 ml), zu. Nach 4 h Rühren und Stehenlassen über Nacht wurde das Lösungsmittel abgezogen und Rückstand mittels präparativer HPLC aufgereinigt. Die produktenthaltenden Fraktionen wurden vereinigt, vom Acetonitril befreit und gefriergetrocknet. Es wurden 24 mg der gewünschten Verbindung erhalten. LCMS-Rt: 1,23 min [M+H]⁺: 524,0

### Beispiel 2

### 1-(3-tert-Butyl-4-methoxy-5-morpholin-4-yl-phenyl)-2-(7-imino-2,3-dimethoxy-imidazo[1,5-b]pyridazin-6-yl)-ethanon als Trifluoressigsäuresalz

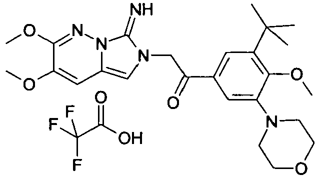

### a) 3,4-Dimethoxy-6-methyl-pyridazin-1-oxid

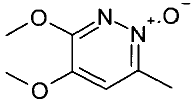

3-Methoxy-6-methyl-4-nitropyridazin-1-oxid (1 g) wurde analog Beispiel 1g) umgesetzt und aufgearbeitet. Eine Chromatographie des Rohprodukts war nicht notwendig. Dabei wurden als Lösungsmittel Methanol (30 ml) und als Base Natriummethylat (als 30%ige Lösung in Methanol, 1,1 ml) eingesetzt. Ausbeute: 900 mg LCMS-Rt: 0,29 min [M+H]⁺: 171,1

### b) Methansulfonsäure-(5,6-dimethoxy-pyridazin-3-yl)-methylester

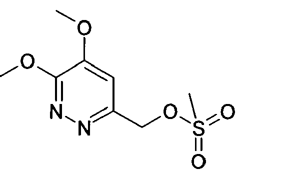

3,4-Dimethoxy-6-methyl-pyridazin-1-oxid (880 mg) wurde analog der Sequenz Beispiel 1 h-1 i) in die Titelverbindung übergeführt. Ausbeute: 690 mg
LCMS-Rt: 0,71 min [M+H]⁺: 249,0

### c) C-(5,6-Dimethoxy-pyridazin-3-yl)-methylamin-Trifluoressigsäuresalz

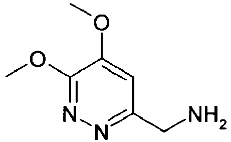

Methansulfonsäure-(5,6-dimethoxy-pyridazin-3-yl)-methylester (690 mg) wurde in Chloroform (10 ml) gelöst und zu einer Urotropin-Lösung (390 mg in 20 ml Chloroform) bei 0 °C getropft. Danach wurde das Kältebad entfernt, 1,5 h bei RT und dann 5 h bei 40°C gerührt. Nach Stehen übers Wochenende wurde erneut 5 h bei 40 °C gerührt. Dann wurde das Lösungsmittel abgezogen, der Rückstand mit Methanol (40 ml) aufgenommen, konzentrierte Salzsäure (1,2 ml) zugegeben und 30 min bei RT gerührt. Dann wurde zur Trockne gebracht, der Rückstand mit Wasser/Acetonitril aufgenommen und gefriergetrocknet. Es wurden 1,2 g des Rohprodukts erhalten. 184 mg davon wurden mittels präparativer HPLC gereinigt. Die Produktent haltenden Fraktionen wurden vereinigt, vom Acetonitril befreit und gefriergetrocknet. Es wurden 40 mg der gewünschten Verbindung erhalten.
LCMS-Rt: 0,20 min [M+H]⁺: 170,1

### d) 2,3-Dimethoxy-imidazo[1,5-b]pyridazin-7-ylamin-Trifluoressigsäuresalz

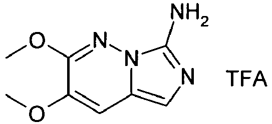

C-(5,6-Dimethoxy-pyridazin-3-yl)-methylamin-Trifluoressigsäuresalz (37 mg) wurde in einer Mischung aus Ethanol und Wasser (3,75/0,75 ml) unter Rühren bei RT vorgelegt. Danach tropfte man Bromcyan-Lösung (0,1 ml; 5 M in ACN) vorsichtig zu und rührte 3 h bei RT. Dann wurde weitere Bromcyan-Lösung (0,1 ml) zugegeben und über Nacht stehen gelassen. Nach erneuter Zugabe von Bromcyan-Lösung (0,1 ml) wurde 4 h gerührt, übers Wochenende stehen gelassen und dann 2 h auf 40°C erwärmt. Anschließend wurde das Lösungsmittel abgezogen und der Rückstand mittels präparativer HPLC gereinigt. Die Produktent haltenden Fraktionen wurden vereinigt, vom Acetonitril befreit und gefriergetrocknet. Es wurden 18 mg der gewünschten Verbindung erhalten. LCMS-Rt: 0,64 min [M+H]⁺: 195,1

### e) 1-(3-tert-Butyl-4-methoxy-5-morpholin-4-yl-phenyl)-2-(7-imino-2,3-dimethoxy-imidazo[1,5-b]pyridazin-6-yl)-ethanon als Trifluoressigsäuresalz

2,3-Dimethoxy-imidazo[1,5-b]pyridazin-7-ylamin-Trifluoressigsäuresalz (15 mg) und 2-Brom-1-(3-tert-butyl-4-methoxy-5-morpholin-4-yl-phenyl)-ethanon (20 mg; Hergestellt wie in WO 2004/078721 beschrieben) wurden analog Beispiel 11) miteinander umgesetzt, aufgearbeitet und gereinigt. Es wurden 27 mg der Titelverbindung erhalten.
LCMS-Rt: 1,28 min [M+H]⁺: 484,4

### Beispiel 3

### 1-(3-tert-Butyl-4-methoxy-5-morpholin-4-yl-phenyl)-2-(2,3-diethoxy-7-imino-imidazo[1,5-b]pyridazin-6-yl)-ethanon als Trifluoressigsäuresalz

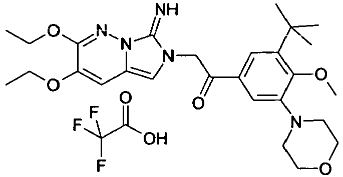

2,3-Diethoxy-imidazo[1,5-b]pyridazin-7-ylamin-Trifluoressigsäuresalz [Beispiel 1k), 15 mg] und 2-Brom-1-(3-tert-butyl-4-methoxy-5-morpholin-4-yl-phenyl)-ethanon (27 mg; Hergestellt wie in WO 2004/078721 beschrieben) wurden analog Beispiel 1l) miteinander umgesetzt, aufgearbeitet und gereinigt. Es wurden 27 mg der Titelverbindung erhalten. LCMS-Rt: 1,38 min [M+H]⁺: 512,3

### Beispiel 4

### 1-(3-tert-Butyl-4-methoxy-5-morpholin-4-yl-phenyl)-2-[7-imino-2-methoxy-3-(2,2,2-trifluor-ethoxy)-imidazo[1,5-b]pyridazin-6-yl]-ethanon als Trifluoressigsäuresalz

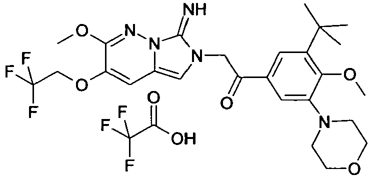

### a) 3-Methoxy-6-methyl-4-(2,2,2-trifluorethoxy)-pyridazin-1-oxid

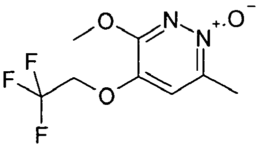

2,2,2-Trifluorethanol (3 ml) wurde bei RT vorgelegt und unter Eiskühlung portionsweise mit Natriumhydrid (403 mg) versetzt. Danach erwärmte man das Reaktionsgemisch 2 h auf 55°C und tropfte dann 3-Methoxy-6-methyl-4-nitropyridazin-1-oxid-Lösung (500 mg, in 3 ml 2,2,2-Trifluorethanol gelöst) zu. Nach 1 h Rühren bei RT wurde 2,5 h auf 55°C erwärmt. Nach Stehen über Nacht bei RT wurde mit Wasser und DCM versetzt. Nach Abtrennen der DCM-Phase wurde die wässrige Phase noch dreimal mit DCM extrahiert und die vereinigten DCM-Phasen über Natriumsulfat getrocknet, filtriert und eingeengt. Es wurden 571 mg der Titelverbindung erhalten. LCMS-Rt: 0,80 min [M+H]⁺: 239,1

### b) C-[6-Methoxy-5-(2,2,2-trifluorethoxy)-pyridazin-3-yl]-methylamin als Trifluoressigsäuresalz

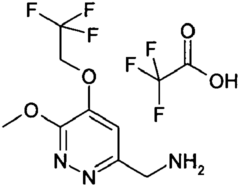

3-Methoxy-6-methyl-4-(2,2,2-trifluorethoxy)-pyridazin-1-oxid (570 mg) wurde analog der Sequenz Beispiel 1h-1j) in die Titelverbindung übergeführt. Es wurden wurden 210 mg der Titelverbindung erhalten. LCMS-Rt: 0,64 min [M+H]⁺: 238,1

### c) 2-Methoxy-3-(2,2,2-trifluorethoxy)-imidazo[1,5-b]pyridazin-7-ylamin als Hydrobromid

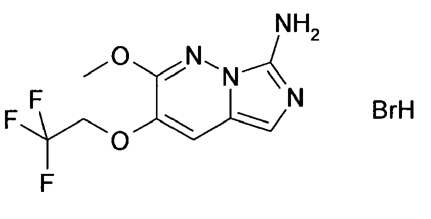

C-[6-Methoxy-5-(2,2,2-trifluorethoxy)-pyridazin-3-yl]-methylamin-Trifluoressigsäure-salz204 mg) wurde in einer Mischung aus Ethanol / Wasser (15/3 ml) vorgelegt. Danach tropfte man Bromcyan-Lösung (62 mg in Ethanol/Wasser 3,75/0,75 ml gelöst) zu. Nach 2 h Rühren bei RT wurde die gleiche Menge Bromcyan erneut zugetropft. Nach 4 h Rühren bei RT ließ man über Nacht stehen und rührte dann weitere 4 h. Anschließend gab man weiteres Bromcyan (60 mg in Ethanol/Wasser 1,88/0,38 ml gelöst) zu. Nach 3 h Rühren bei RT ließ man erneut über Nacht stehen und zog dann das Lösungsmittel ab. Der Rückstand wurde über Kieslegel ( 25 g Kartusche, DCM/Methanol-Gradient) aufgereinigt. Die Substanz enthaltenden Fraktionen wurden vereinigt und zur Trockne gebracht. Neben 62 mg Edukt wurden 20 mg der Titelverbindung erhalten. LCMS-Rt: 0,86 min [M+H]⁺: 263,0

### d) 1-(3-tert-Butyl-4-methoxy-5-morpholin-4-yl-phenyl)-2-[7-imino-2-methoxy-3-(2,2,2-trifluor-ethoxy)-imidazo[1,5-b]pyridazin-6-yl]-ethanon als Trifluoressigsäuresalz

2-Methoxy-3-(2,2,2-trifluorethoxy)-imidazo[1,5-b]pyridazin-7-ylamin-Hydrobromid (18 mg) und 2-Brom-1-(3-tert-butyl-4-methoxy-5-morpholin-4-yl-phenyl)-ethanon (22 mg; Hergestellt wie in WO 2004/078721 beschrieben) wurden analog Beispiel 1l) miteinander umgesetzt, aufgearbeitet und gereinigt. Es wurden 31 mg der Titelverbindung erhalten. LCMS-Rt: 1,39 min [M+H]⁺: 552,2

### Beispiel 5

### 2-(2,3-Diethoxy-7-imino-imidazo[1,5-b]pyridazin-6-yl)-1-[3-methylamino-5-(pentafluorsulfanyl)-phenyl]-ethanon als Trifluoressigsäuresalz

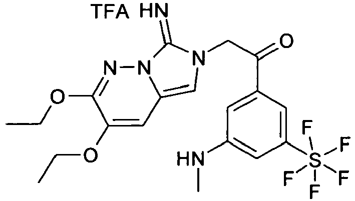

### a) N-Methoxy-N-methyl-3-(pentafluorsulfanyl)-5-(2,2,2-trifluor-acetylamino)-benzamid

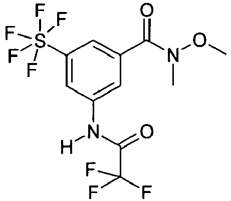

3-Amino-N-methoxy-N-methyl-5-pentafluorsulfanyl-benzamid [Beispiel 1 c); 1,45 g] wurde in Methylenchlorid (15 ml) gelöst und unter Rühren Triethylamin (0,8 ml) gefolgt von Trifluoressigsäureanhydrid (0,85 ml) unter Feuchtigkeitsausschluss zugesetzt. Nach 3 h Rühren bei RT und Stehen über Nacht wurden Wasser und gesättigte Natriumhydrogencarbonat-Lösung zugegeben, die Phasen getrennt und die Methylenchlorid-Phase noch dreimal mit Wasser gewaschen, über Magnesiumsulfat getrocknet, filtriert und eingeengt. Das erhaltene Produkt (1,75 g) wurde ohne weitere Reinigung in der nächsten Stufe eingesetzt.
LCMS-Rt: 1,53 min [M+H]⁺: 403,0

### b) N-(3-Acetyl-5-pentafluorsulfanyl-phenyl)-2,2,2-trifluor-acetamid

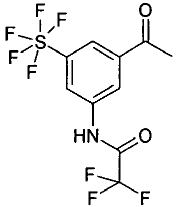

N-Methoxy-N-methyl-3-(pentafluorsulfanyl)-5-(2,2,2-trifluor-acetylamino)-benzamid (1,65 g) wurde in THF (25 ml) gelöst. Bei 0°C wurde unter Rühren Lithium-bis(trimethylsilyl)-amid (0,9 ml) zugegeben. Nach 30 min wurde Methylmagnesiumbromid (3,5 ml, 3 M in Diethylether) zugetropft. Nach beendeter Zugabe wurde das Eisbad entfernt und 2 h bei RT gerührt. Unter Kühlung wurden dann 1 N Salzsäure, Wasser und EE zugegeben. Nach Abtrennen der organischen Phase wurde die wässrige Phase noch zweimal mit EE extrahiert. Die vereinigten EE-Phasen wurden mit Magnesiumsulfat getrocknet, filtriert und eingeengt. Das Rohprodukt ist ein Gemisch aus N-(3-Acetyl-5-pentafluorsulfanyl-phenyl)-2,2,2-trifluor-acetamid und 1-[3-Amino-5-(pentafluorsulfanyl)-phenyl]-ethanon, so dass das Rohprodukt (1,3 g) in Methylenchlorid (60 ml) aufgenommen und mit Triethylamin (155 µl) versetzt wurde. Danach wurde unter Rühren Trifluoressigsäureanhydrid (160 µl) zugegeben. Nach 3 h Rühren bei RT wurden Wasser und gesättigte Natriumhydrogencarbonat-Lösung zugegeben, die Phasen getrennt und die DCM-Phase noch dreimal mit Wasser gewaschen. Die DCM-Phase wurde mit Magnesiumsulfat getrocknet, filtriert und eingeengt. Es wurden 1,3 g der Titelverbindung erhalten. LCMS-Rt: 1,61 min [M+H]⁺: 358,0

### c) N-[3-Acetyl-5-(pentafluorsulfanyl)-phenyl]-2,2,2-trifluor-N-methyl-acetamid

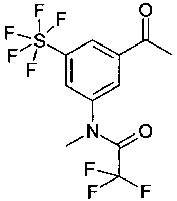

In einem Mikrowelleneinsatz wurde N-(3-Acetyl-5-pentafluorsulfanyl-phenyl)-2,2,2-trifluor-acetamid (0,25 g) in absolutem Dimethoxyethan (7,5 ml) gelöst, gepulvertes Kaliumcarbonat zugegeben und mit Jodmethan (80 µl) versetzt. Anschließend wurde in der Mikrowelle 40 min auf 100°C erhitzt. Nachdem weiteres N-(3-Acetyl-5-pentafluorsulfanyl-phenyl)-2,2,2-trifluor-acetamid (4 x 250 mg) in beschriebener Weise umgesetzt worden war, wurden die fünf Ansätze gemeinsam aufgearbeitet, wobei vom Kaliumcarbonat unter Eiskühlung in 1 N Salzsäure dekantiert wurde. Nach mehrmaligem Waschen des Kaliumcarbonat-Rückstands mit Dimethoxyethan wurde die wässrige Phase fünfmal mit Essigester extrahiert. Die vereinigten Extrakte wurden über Magnesiumsulfat getrocknet, filtriert und eingeengt. Der Rückstand wurde mittels präparativer HPLC gereinigt. Die Produkt enthaltenden Fraktionen wurden vereinigt, vom Acetonitril befreit und fünfmal mit Essigester extrahiert. Die vereinigten Extrakte wurden über Magnesiumsulfat getrocknet, filtriert und eingeengt. Es wurden 1,03 g der gewünschten Verbindung erhalten.
LCMS-Rt: 1,62 min [M+H]⁺: 372,0

### d) N-[3-(2-Brom-acetyl)-5-(pentafluorsulfanyl)-phenyl]-2,2,2-trifluor-N-methyl-acetamid

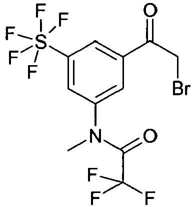

N-[3-Acetyl-5-(pentafluorsulfanyl)-phenyl]-2,2,2-trifluor-N-methyl-acetamid (1,03 g) wurde in einem Gemisch aus Methanol (20 ml) und THF (20 ml) gelöst. Unter Rühren wurde Phenyl-trimethylammoniumtribromid (1,05 g) zugegeben. Nach 5 h Rühren bei RT wurde über Nacht stehen gelassen, dann weiteres Phenyl-trimethylammoniumtribromid (100 mg) zugegeben und 2 h auf 60°C erwärmt. Nach Erkalten wurde das Reaktionsgemisch in 2 N Schwefelsäure gegeben und 10 min gerührt. Dann wurde die wässrige Phase dreimal mit EE extrahiert. Die vereinigten organischen Phasen wurden über Magnesiumsulfat getrocknet und nach Abfiltrieren des Trockenmittels im Vakuum zur Trockne gebracht. Es wurden 1,2 g der Titelverbindung erhalten, die ausreichende Reinheit für die nächsten Umsetzungen hatte. LCMS-Rt: 1,72 min [M+H]⁺: 449,9

### e) 2-Brom-1-[3-methylamino-5-(pentafluorsulfanyl)-phenyl]-ethanon

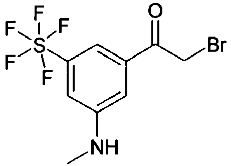

N-[3-(2-Brom-acetyl)-5-(pentafluorsulfanyl)-phenyl]-2,2,2-trifluor-N-methyl-acetamid (01.075; 1,2 g) wurde mit Wasser (15 ml) versetzt und unter Rühren und Eiskühlung konzentrierte Schwefelsäure (15 ml) zugetropft. Es wurde auf 80°C erwärmt und 7 h bei dieser Temperatur gerührt. Nach Erkalten wurde das Reaktionsgemisch langsam in ein Gemisch aus 10 N Natriumhydroxid-Lösung und EE gegeben und die wässrige Phase fünfmal mit EE extrahiert. Die vereinigten organischen Phasen wurden über Magnesiumsulfat getrocknet und nach Abfiltrieren des Trockenmittels im Vakuum zur Trockne gebracht. Der Rückstand wurde mittels präparativer HPLC gereinigt. Die jeweils sauberen Produktfraktionen wurden vereinigt, im Vakuum vom Acetonitril befreit, mit Natriumhydrogencarbonat neutralisiert und dreimal mit EE extrahiert. Die vereinigten organischen Phasen wurden über Magnesiumsulfat getrocknet und nach Abfiltrieren des Trockenmittels im Vakuum zur Trockne gebracht. Es wurden 420 mg der Titelverbindung isoliert. LCMS-Rt: 1,64 min [M+H]⁺: 354,0

### f) 2-(2,3-Diethoxy-7-imino-imidazo[1,5-b]pyridazin-6-yl)-1-[3-methylamino-5-(pentafluorsulfanyl)-phenyl]-ethanon als Trifluoressigsäuresalz

2,3-Diethoxy-imidazo[1,5-b]pyridazin-7-ylamin-Trifluoressigsäuresalz [Beispiel 1k), 10 mg] und 2-Brom-1-[3-methylamino-5-(pentafluorsulfanyl)-phenyl]-ethanon (18 mg) wurden analog Beispiel 11) miteinander umgesetzt, aufgearbeitet und gereinigt. Zur Nachreinigung wurde nach der präparativen HPLC noch einmal über Kieselgel chromatographiert (2 g Kartusche; DCM/Methanol-Gradient). Nach Vereinigung der sauberen Fraktionen wurde zur Trockne gebracht. Der Rückstand wurde mir ACN/Wasser (+ 0,05 % TFA) aufgenommen und gefriergetrocknet. Es wurden 4 mg der Titelverbindung erhalten. LCMS-Rt: 1,29 min [M+H]⁺: 491,1

### Pharmakologische Beispiele

### PAR 1 Bestimmungsmethode: Hemmung der PAR1 mediierten Thrombozytenaggregation

Die pharmakologische Testung der Substanzen erfolgte in der durch TRAP (Thrombinrezeptor aktivierendes Peptid) induzierten Thrombozytenaggregation im 96-well Format. Dazu wurde von gesunden freiwilligen Spendern Blut in 20 ml Spritzen abgenommen, in denen 2 ml 3,13 %-ige Natriumcitratlösung vorgelegt war. Nach einer 20-minütigen Zentrifugation bei 150 x g wurde das Plättchenreiche Plasma (PRP) abgetrennt und mit 1 µl PGE1-Lösung (500 µg/ml in Ethanol) / ml PRP versetzt. Nach 5 Minuten Inkubation bei Raumtemperatur wurde 15 Minuten bei 120 x g zentrifugiert um die Leukozyten zu entfernen. Das Leukozytenfreie PRP wurde in 5 ml Portionen in 15 ml PP Röhrchen überführt und 15 Minuten bei 360xg abzentrifugiert, um die Plättchen zu pelletieren. Anschließend wurde das Plasma dekantiert und das Plättchensediment aus 5 ml PRP in 1 ml Tyrode (120 mM NaCl, 2,6 mM KCl, 12 mM NaHCO₃, 0,39 mM NaH₂PO₄ x H₂O, 10 mM HEPES, 0,35% BSA, 5,5 mM Glukose, pH 7,4) resuspendiert und mit Tyrode auf eine Plättchenzahl von 3x10⁵ / Mikroliter (µL) eingestellt. 13 ml dieser Zellsuspension wurde dann mit 866 µL 10 mM CaCl₂-Lösung versetzt und 120 µL davon pro well einer 96-well-Platte pipettiert, in dem 15 µL der zu testenden Substanz vorgelegt waren. Nach 30 Minuten Inkubation bei Raumtemperatur im Dunklen wurden 15 µL einer TRAP-Lösung (70-100 µM) als Agonist zugegeben und in einem SpectraMax 340 bei 650 nm über 20 Minuten bei 37° C unter Schütteln eine Kinetik aufgezeichnet. Die Flächen unter den Kurven von Negativkontrolle (Tyrode/ DMSO) und Positivkontrolle (15 µl Agonist /DMSO) wurden berechnet und die Differenz als 100 % Wert festgelegt. Die zu testenden Substanzen wurden in Doppelbestimmung als Verdünnungsreihen pipettiert, ebenfalls die AUC jeder Substanzkonzentration bestimmt und die % Hemmung der AUC gegen die Kontrolle errechnet. Anhand der % Hemmung wurde mit Hilfe nichtlinearer Regressionsanalyse gemäß der 4 Parameter-Gleichung die IC50 berechnet.

Tabelle 1 zeigt die Ergebnisse.

**Tabelle 1:**

| Verbindung aus Beispiel | Hemmung der Thrombozytenaggregation IC₅₀ [mikro M] | Verbindung aus Beispiel | Hemmung der Thrombozytenaggregation IC₅₀ [mikro M] |
|---|---|---|---|
| 1 | 0,005 | 3 | 0,004 |
| 4 | 0,18 | | |

## Patentansprüche

1. Verbindung der Formel I 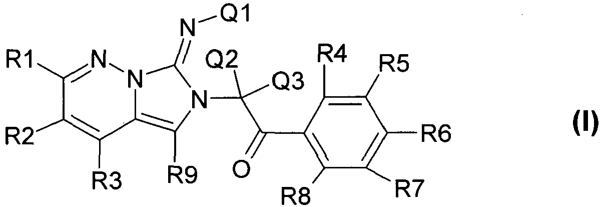 und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
Q1, Q2 und Q3 gleich oder verschieden sind und unabhängig voneinander für ein Wasserstoffatom oder -(C₁-C₆)-Alkyl stehen, wobei in Alkyl die Wasserstoffatome ganz oder teilweise durch Fluor ersetzt sein können,
R1, R2 und R3 gleich oder verschieden sind und unabhängig voneinander für Wasserstoffatom, -(C₁-C₆)-Alkyl, -(C₃-C₆)-Cycloalkyl, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-Alkylen-C(O)-R11, -(C₀-C₄)-Alkylen-N(R11)-R12, -(C₀-C₄)-Alkylen-N(R11)-C(O)-R12, Halogen, OH, -CN, -(C₁-C₆)-Alkylen-O-(C₁-C₆)-Alkyl, -O-(C₁-C₆)-Alkylen-O-(C₁-C₆)-Alkyl oder -O-(C₁-C₄)-Alkylen-(C₃-C₆)-Cycloalkyl, stehen,
wobei Alkyl, Alkylen und Cycloalkyl jeweils unsubstituiert oder ein-, zwei- oder dreifach unabhängig voneinander durch -(C₁-C₄)-Alkyl, OH oder -O-(C₁-C₆)-Alkyl substituiert sind, oder
wobei in Alkyl, Alkylen oder Cycloalkyl die Wasserstoffatome ganz oder teilweise durch Fluor ersetzt sein können,
mit der Maßgabe, das mindestens ein R1, R2 oder R3 nicht Wasserstoffatom ist,
R11 und R12 unabhängig voneinander für Wasserstoffatom, -(C₁-C₆)-Alkyl oder -(C₃-C₆)-Cycloalkyl stehen,
R4, R5, R6, R7 und R8 gleich oder verschieden sind und unabhängig voneinander für Wasserstoffatom, -(C₁-C₆)-Alkyl, -(C₃-C₆)-Cycloalkyl, OH, -CN, -O-(C₁-C₈)-Alkyl, -O-(C₃-C₆)-Cycloalkyl, -(C₀-C₄)-Alkylen-(CO)-N(R21)-R22, -(C₀-C₄)-Alkylen-C(O)-O-R21, Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -(C₀-C₆)-Alkylen-O-(C₁-C₆)-Alkylen-O-(C₁-C₆)-Alkyl, oder -(C₀-C₆)-Alkylen-O-(C₁-C₄)-Alkylen-(C₃-C₆)-Cycloalkyl stehen,
wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, oder
wobei in Alkyl, Alkylen oder Cycloalkyl die Wasserstoffatome ganz oder teilweise durch Fluor ersetzt sein können,
mit der Maßgabe, das mindestens ein R4, R5, R6, R7 oder R8 nicht Wasserstoffatom ist,
R9 für ein Wasserstoffatom oder -(C₁-C₆)-Alkyl steht, wobei in Alkyl die Wasserstoffatome ganz oder teilweise durch Fluor ersetzt sein können,
R21 und R22 unabhängig voneinander für ein Wasserstoffatom, -(C₁-C₆)-Alkyl oder -(C₃-C₆)-Cycloalkylstehen,
wobei in Alkyl oder Cycloalkyl die Wasserstoffatome ganz oder teilweise durch Fluor ersetzt sein können, oder
R21 und R22 in den Fragmenten "N(R21)-R22" und "N(R21)-C(O)-R22" für einen Ring stehen, ausgewählt aus der Gruppe Pyrrolidinyl, Piperidinyl, Piperazinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können.

2. Verbindung der Formel I gemäß Anspruch 1, wobei
Q1, Q2 und Q3 gleich sind und für jeweils ein Wasserstoffatom stehen, R1, R2 und R3 gleich oder verschieden sind und unabhängig voneinander für Wasserstoffatom, -(C₁-C₄)-Alkyl, -O-CH₂-CF₃ oder -O-(C₁-C₆)-Alkyl stehen, mit der Maßgabe, das mindestens ein R1, R2 oder R3 nicht Wasserstoffatom ist,
R4 und R8 jeweils für ein Wasserstoffatom steht,
R5, R6 und R7 gleich oder verschieden sind und unabhängig voneinander für Wasserstoffatom, -(C₁-C₆)-Alkyl, -O-(C₁-C₄)-Alkyl, -SF₅ oder -N(R21)-R22 stehen,
mit der Maßgabe, das mindestens ein R5, R6 oder R7 nicht Wasserstoffatom ist, und
R9 für Wasserstoffatom steht,
R21 und R22 unabhängig voneinander für Wasserstoffatom oder -(C₁-C₄)-Alkyl stehen, oder
R21 und R22 in dem Fragment "N(R21)-R22" für einen Ring stehen, ausgewählt aus der Gruppe Pyrrolidinyl, Piperidinyl, Piperazinyl, Imidazolyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl.

3. Verbindung der Formel I gemäß Anspruch 1, wobei die Verbindung der Formel I ausgewählt ist aus der Gruppe 1-(3-tert-Butyl-4-methoxy-5-morpholin-4-yl-phenyl)-2-(7-imino-2,3-dimethoxy-imidazo[1,5-b]pyridazin-6-yl)-ethanon, 1-(3-tert-Butyl-4-methoxy-5-morpholin-4-yl-phenyl)-2-(2,3-diethoxy-7-imino-imidazo[1,5-b]pyridazin-6-yl)-ethanon, N-{3-[2-(2,3-Diethoxy-7-imino-imidazo[1,5-b]pyridazin-6-yl)-acetyl]-5-pentafluorsulfanyl-phenyl}-acetamid, 1-(3-tert-Butyl-4-methoxy-5-morpholin-4-yl-phenyl)-2-[7-imino-2-methoxy-3-(2,2,2-trifluoro-ethoxy)-imidazo[1,5-b]pyridazin-6-yl]-ethanon, 2-(2,3-Diethoxy-7-imino-imidazo[1,5-b]pyridazin-6-yl)-1-(5-methylamino-3-pentafluorsulfanyl-phenyl)-ethanon oder 2-(2,3-Diethoxy-7-imino-imidazo[1,5-b]pyridazin-6-yl)-1-[3-methylamino-5-(pentafluorsulfanyl)-phenyl]-ethanon.

4. Arzneimittel, **gekennzeichnet durch** einen wirksamen Gehalt an mindestens einer Verbindung der Formel I gemäß einem oder mehreren der Ansprüche 1 bis 3 zusammen mit einem pharmazeutisch geeigneten und physiologisch verträglichen Trägerstoff, Zusatzstoff und/oder anderen Wirk- und Hilfsstoffen.

5. Verwendung der Verbindung der Formel I gemäß einem oder mehreren der Ansprüche 1 bis 3 zur Herstellung eines Arzneimittels zur Prophylaxe, Sekundärprevention und Therapie all solcher Erkrankungen, die die mit Thrombosen, Embolien, Hyperkoagulabilität, fibrotischen Veränderungen oder entzündlichen Erkrankungen einhergehen.

6. Verwendung gemäß Anspruch 5, **dadurch gekennzeichnet, dass** es sich um Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder Eingriffen, die zu einem Kontakt des Blutes mit Fremdoberflächen führen wie bei Dialysepatienten und Patienten mit Verweilkathetern oder um die disseminierte intravaskuläre Koagulation, Sepsis und anderen intravaskulären Ereignissen, die mit einer Entzündung einhergehen, Atherosklerose, Diabetes und dem metabolischen Syndrom und deren Folgen, Tumorwachstum und Tumormetastasierung, entzündliche und degenerative Gelenkserkrankungen wie der rheumatoiden Arthritis und der Arthrose, Störungen des hämostatischen Systems wie Fibrinablagerungen, fibrotische Veränderungen der Lunge wie die chronische obstruktive Lungenerkrankung, das adult respiratory distress syndrom oder Fibrinablagerungen des Auges nach Augenoperationen oder Verhinderung und/oder Behandlung von Narbenbildung.

7. Verfahren zur Herstellung der Verbindung der Formel I gemäß einem oder mehreren der Ansprüche 1 bis 3, **dadurch gekennzeichnet, dass** man
a) eine Verbindung der Formel II 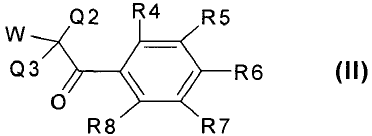 wobei R4, R5, R6, R7, R8, Q2 und Q3 wie in Formel I definiert sind und W für Chlorid, Bromid, Mesylat oder Tosylat steht, mit einer Verbindung der Formel III, 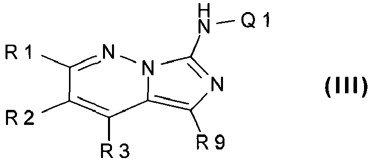 wobei R1, R2, R3, R9 und Q1 wie in Formel I definiert sind, mit oder ohne Basenzugabe in einem Lösungsmittel zu einer Verbindung der Formel I umsetzt, oder
b) eine Verbindung der Formel VII, 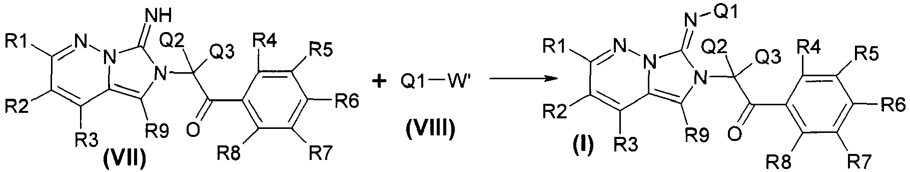 wobei R1, R2, R3, R4, R5, R6, R7, R8, R9, Q1, Q2 und Q3 wie in Formel I definiert sind, mit einer Verbindung Q1-W', wobei W' Chlorid, Bromid, Tosylat, Mesylat, Methylsulfat oder eine ähnliche gute Fluchtgruppe bedeutet, mit oder ohne Basenzugabe zu einer Verbindung der Formel I umsetzt, oder
c) eine Verbindung der Formel XXVI, 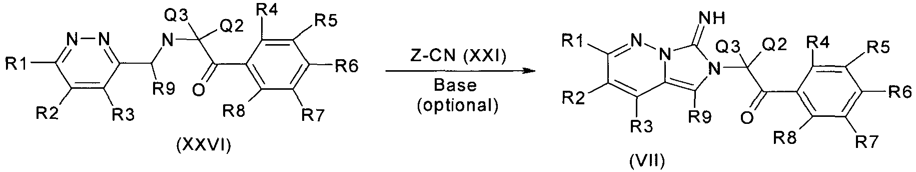 wobei R1, R2, R3, R4, R5, R6, R7, R8, R9, Q1, Q2 und Q3 wie in Formel I definiert sind, mit einer Verbindung Z-CN, wobei Z eine gute Fluchtgruppe wie Tosylat, Chlorid oder Bromid bedeutet, mit oder ohne Basenzugabe zu einer Verbindung der Formel VII umsetzt, oder
d) eine Verbindung der Formel XXVII, 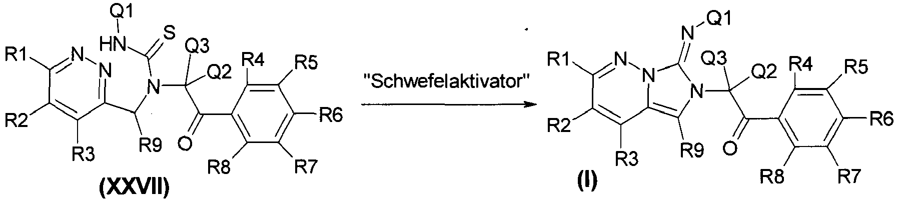 wobei R1, R2, R3, R4, R5, R6, R7, R8, R9, Q1, Q2 und Q3 wie in Formel I definiert sind, mit einem Schwefelaktivator zu einer Verbindung der Formel I umsetzt, oder
e) die nach den Verfahren a) bis d) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
f) eine nach den Verfahren a) bis d) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

## Claims

1. A compound of the formula I 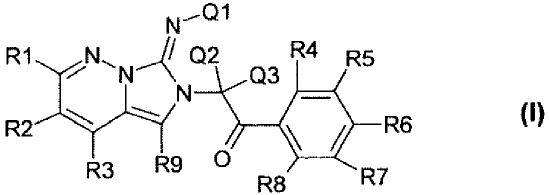 and/or any stereoisomeric or tautomeric forms of the compound of the formula I and/or mixtures of these forms in any ratio, and/or a physiologically compatible salt of the compound of the formula I, where
Q1, Q2 and Q3 are the same or different and are each independently a hydrogen atom or -(C₁-C₆)-alkyl, where some or all of the hydrogen atoms in alkyl may be replaced by fluorine,
R1, R2 and R3 are the same or different and are each independently a hydrogen atom, -(C₁-C₆)-alkyl, -(C₃-C₆)-cycloalkyl, -O-(C₁-C₈)-alkyl, -O-(C₃-C₆)-cycloalkyl, -(C₀-C₄)-alkylene-C(O)-N(R11)-R12, - (C₀-C₄)-alkylene-C(O)-O-R11, -(C₀-C₄)-alkylene-C(O)-R11, -(C₀-C₄)-alkylene-N(R11 )-R12, -(C₀-C₄)-alkylene-N(R11)-C(O)-R12, halogen, OH, -CN, -(C₁-C₆)-alkylene-O-(C₁-C₆)-alkyl, -O-(C₁-C₆)-alkylene-O-(C₁-C₆)-alkyl or -O-(C₁-C₄)-alkylene-(C₃-C₆)-cycloalkyl where alkyl, alkylene and cycloalkyl are each unsubstituted or mono-, di- or trisubstituted independently by -(C₁-C₄)-alkyl, OH or -O-(C₁-C₆)-alkyl, or
where some or all of the hydrogen atoms in alkyl, alkylene or cycloalkyl may be replaced by fluorine,
with the proviso that at least one R1, R2 or R3 is not a hydrogen atom,
R11 and R12 are each independently a hydrogen atom, -(C₁-C₆)-alkyl or -(C₃-C₆)-cycloalkyl,
R4, R5, R6, R7 and R8 are the same or different and are each independently a hydrogen atom, -(C₁-C₆)-alkyl, -(C₃-C₆)-cycloalkyl, OH, -CN, -O-(C₁-C₈)-alkyl, -O-(C₃-C₆)-cycloalkyl, -(C₀-C₄)-alkylene-(CO)-N(R21)-R22, -(C₀-C₄)-alkylene-C(O)-O-R21, halogen, -SF₅, -(C₀-C₄)-alkylene-C(O)-R21, -(C₀-C₄)-alkylene-N(R21)-R22, -(C₀-C₄)-alkylene-N(R21)-C(O)-R22, -(C₁-C₆)-alkylene-O-(C₁-C₆)-alkyl, -(C₀-C₆)-alkylene-O-(C₁-C₆)-alkylene-O-(C₁-C₆)-alkyl or -(C₀-C₆)-alkylene-O-(C₁-C₄)-alkylene-(C₃-C₆)-cycloal kyl,
where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl or -O-(C₃-C₆)-cycloalkyl, or where some or all of the hydrogen atoms in alkyl, alkylene or cycloalkyl may be replaced by fluorine,
with the proviso that at least one R4, R5, R6, R7 or R8 is not a hydrogen atom,
R9 is a hydrogen atom or -(C₁-C₆)-alkyl, where some or all of the hydrogen atoms in alkyl may be replaced by fluorine,
R21 and R22 are each independently a hydrogen atom, -(C₁-C₆)-alkyl or -(C₃-C₆)-cycloalkyl,
where some or all of the hydrogen atoms in alkyl or cycloalkyl may be replaced by fluorine, or
R21 and R22 in the "N(R21)-R22" and "N(R21)-C(O)-R22" fragments represent a ring selected from the group of pyrrolidinyl, piperidinyl, piperazinyl, morpholinyl, thiomorpholinyl, pyrrolidine-2.5-dionyl, piperidine-2,6-dionyl, piperazine-2,6-dionyl, morpholine-3,5-dionyl, pyrrolidin-2-onyl, piperidin-2-onyl, piperazin-2-onyl and morpholin-3-onyl, where the ring is unsubstituted or mono- or disubstituted independently by -(C₁-C₄)-alkyl, -(C₃-C₆)-cycloalkyl, OH, -O-(C₁-C₆)-alkyl or -O-(C₃-C₆)-cycloalkyl, where some or all of the hydrogen atoms in alkyl or cycloalkyl may be replaced by fluorine.

2. A compound of the formula I as claimed in claim 1, wherein
Q1, Q2 and Q3 are the same and are each independently a hydrogen atom,
R1, R2 and R3 are the same or different and are each independently a hydrogen atom, -(C₁-C₄)-alkyl, -O-CH₂-CF₃ or -O-(C₁-C₆)-alkyl, with the proviso that at least one R1, R2 or R3 is not a hydrogen atom,
R4 and R8 are each a hydrogen atom,
R5, R6 and R7 are the same or different and are each independently a hydrogen atom, -(C₁-C₆)-alkyl, -O-(C₁-C₄)-alkyl, -SF₅ or -N(R21)-R22,
with the proviso that at least one R5, R6 or R7 is not a hydrogen atom, and
R9 is a hydrogen atom,
R21 and R22 are each independently a hydrogen atom or -(C₁-C₄)-alkyl, or
R21 and R22 in the "N(R21)-R22" fragment are each a ring selected from the group of pyrrolidinyl, piperidinyl, piparazinyl, imidazolyl, morpholinyl, thiomorpholinyl, pyrrolidine-2,5-dionyl, piperidine-2,6-dionyl, piperazine-2,6-dionyl, morpholine-3,5-dionyl, pyrrolidin-2-onyl, piperidin-2-onyl, piperazin-2-onyl and morpholin-3-onyl.

3. A compound of the formula I according to claim 1, wherein the compound of the formula I is selected from the group of 1-(3-tert-butyl-4-methoxy-5-morpholin-4-ylphenyl)-2-(7-imino-2,3-dimethoxyimidazo[1,5-b]pyridazin-6-yl)ethanone, 1-(3-tert-butyl-4-methoxy-5-morpholin-4-ylphenyl)-2-(2,3-diethoxy-7-iminoimidazo-[1,5-b]pyridazin-6-yl)ethanone, N-{3-[2-(2,3-diethoxy-7-iminoimidazo[1,5-b]pyridazin-6-yl)acetyl]-5-pentafluorosulfanylphenyl}acetamide, 1-(3-tert-butyl-4-methoxy-5-morphol in-4-ylphenyl)-2-[7-imino-2-methoxy-3-(2,2,2-trifluoroethoxy)imidazo[1,5-b]pyridazin-6-yl]ethanone, 2-(2,3-diethoxy-7-iminoimidazo[1,5-b]pyridazin-6-yl)-1-(5-methylamino-3-pentafluorosulfanylphenyl)ethanone or 2-(2,3-diethoxy-7-iminoimidazo[1,5-b]pyridazin-6-yl)-1-[3-methylamino-5-(pentafluoro-sulfanyl)phenyl]ethanone.

4. A medicament, **characterized by** an effective content of at least one compound of the formula I as claimed in one or more of claims 1 to 3 together with a pharmaceutically suitable and physiologically compatible carrier, additive and/or other active ingredients and excipients.

5. The use of the compound of the formula I as claimed in one or more of claims 1 to 3 for producing a medicament for prophylaxis, secondary prevention and treatment of all of those disorders associated with thromboses, embolisms, hypercoagulability, fibrotic changes or inflammatory disorders.

6. The use as claimed in claim 5, which is applied to myocardial infarction, angina pectoris and other types of acute coronary syndrome, stroke, peripheral vascular disorders, deep vein thrombosis, pulmonary embolism, embolic or thrombotic events caused by cardiac arrhythmias, cardiovascular events such as restenosis following revascularization and angioplasty and similar procedures such as stent implantations and bypass operations or reduction of the risk of thrombosis following surgical procedures such as knee and hip joint operations or procedures leading to contact of blood with foreign surfaces, such as for dialysis patients and patients with indwelling catheters or disseminated intravascular coagulation, sepsis and other intravascular events associated with inflammation, atherosclerosis, diabetes and the metabolic syndrome and the sequelae thereof, tumor growth and tumor metastasis, inflammatory and degenerative articular disorders such as rheumatoid arthritis and arthrosis, impairments of the hemostatic system such as fibrin deposits, fibrotic changes in the lung such as chronic obstructive pulmonary disease, adult respiratory distress syndrome or fibrin deposits in the eye following eye operations or prevention and/or treatment of scarring.

7. A process for preparing the compound of the formula I as claimed in one or more of claims 1 to 3, which comprises
a) reacting a compound of the formula II 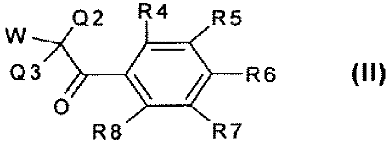 where R4, R5, R6, R7, R8, Q2 and Q3 are each as defined in formula I and W is chloride, bromide, mesylate or tosylate with a compound of the formula III 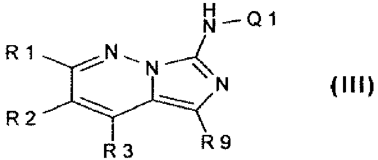 where R1, R2, R3, R9 and Q1 are each as defined in formula I, with or without addition of base, in a solvent to give a compound of the formula I, or
b) reacting a compound of the formula VII, 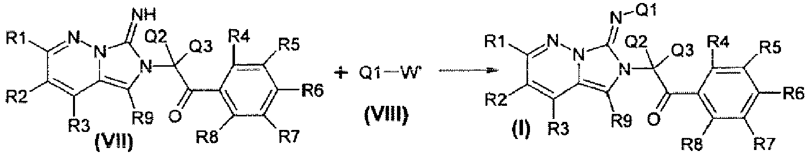 where R1, R2, R3, R4, R5, R6, R7, R8, R9, Q1, Q2 and Q3 are each as defined in formula I with a compound Q1-W' where W' is chloride, bromide, tosylate, mesylate, methylsulfate or a similarly good leaving group, with or without addition of base, to give a compound of the formula I, or
c) reacting a compound of the formula XXVI 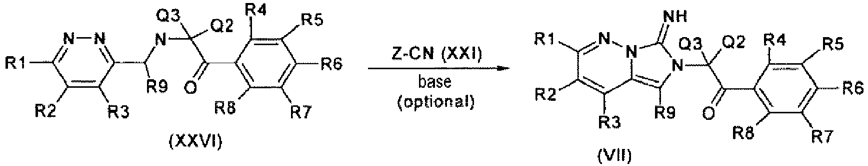 where R1, R2, R3, R4, R5, R6, R7, R8, R9, Q1, Q2 and Q3 are each as defined in formula I with a compound Z-CN where Z is a good leaving group such as tosylate, chloride or bromide, with or without addition of base, to give a compound of the formula VII, or
d) reacting a compound of the formula XXVII 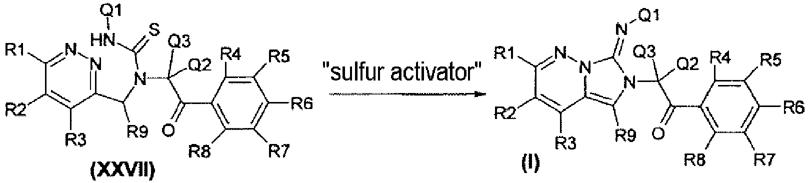 where R1, R2, R3, R4, R5, R5, R7, R8, R9, Q1, Q2 and Q3 are each as defined in formula I with a sulfur activator to give a compound of the formula I, or
e) either isolating the compound of the formula I prepared by methods a) to d) in free form or releasing it from physiologically incompatible salts or, in the case of the presence of acidic or basic groups, converting it to physiologically compatible salts, or
f) separating a compound of the formula I prepared by methods a) to d), or a suitable precursor of the formula I which, owing to its chemical structure, occurs in enantiomeric or diastereomeric forms, into the pure enantiomers or diastereomers by salt formation with enantiomerically pure acids or bases, chromatography on chiral stationary phases or derivatization by means of chiral enantiomerically pure compounds such as amino acids, separation of the diastereomers thus obtained, and elimination of the chiral auxiliary groups.

## Revendications

1. Composé de formule I 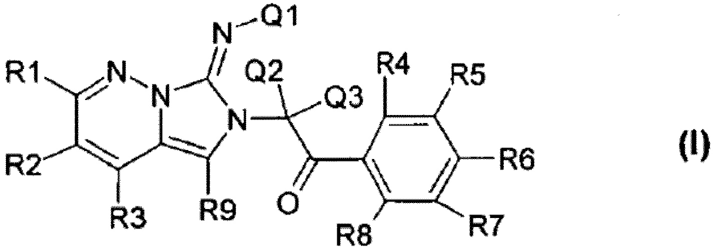 et/ou toutes les formes stéréoisomères ou tautomères du composé de formule I et/ou les mélanges de ces formes en tout rapport et/ou un sel physiologiquement compatible du composé de formule I, dans lequel
Q1, Q2 et Q3 sont identiques ou différents, et représentent indépendamment les uns des autres un atome d'hydrogène ou -alkyle en (C₁-C₆), les atomes d'hydrogène dans l'alkyle pouvant être remplacés en totalité ou en partie par fluor,
R1, R2 et R3 sont identiques ou différents, et représentent indépendamment les uns des autres un atome d'hydrogène, -alkyle en (C₁-C₆), -cycloalkyle en (C₃-C₆), -O-alkyle en (C₁-C₈), -O-cycloalkyle en (C₃-C₆), -alkylène en (C₀-C₄)-C(O)-N(R11)-R12, -alkylène en (C₀-C₄)-C(O)-O-R11,-alkylène en (C₀-C₄)-C(O)-R11, -alkylène en (C₀-C₄)-N(R11)-R12, -alkylène en (C₀-C₄)-N(R11)-C(O)-R12, halogène, OH, -CN, -alkylène en (C₁-C₆)-O-alkyle en (C₁-C₆), -O-alkylène en (C₁-C₆)-O-alkyle en (C₁-C₆) ou -O-alkylène en (C₁-C₄)-cycloalkyle en (C₃-C₆),
alkyle, alkylène et cycloalkyle étant chacun non substitués ou substitués une, deux ou trois fois indépendamment les uns des autres par -alkyle en (C₁-C₄), OH ou -O-alkyle en (C₁-C₆), ou
les atomes d'hydrogène dans alkyle, alkylène ou cycloalkyle pouvant être remplacés en totalité ou en partie par fluor,
à condition qu'au moins un radical parmi R1, R2 et R3 ne représente pas un atome d'hydrogène,
R11 et R12 représentent indépendamment l'un de l'autre un atome d'hydrogène, -alkyle en (C₁-C₆) ou -cycloalkyle en (C₃-C₆), R4, R5, R6, R7 et R8 sont identiques ou différents, et représentent indépendamment les uns des autres un atome d'hydrogène, -alkyle en (C₁-C₆), -cycloalkyle en (C₃-C₆), OH, -CN, -O-alkyle en (C₁-C₈), -O-cycloalkyle en (C₃-C₆), -alkylène en (C₀-C₄)-C(O)-N(R21)-R22, -alkylène en (C₀-C₄)-C(O)-O-R21, halogène, -SF₅, -alkylène en (C₀-C₄)-C(O)-R21, -alkylène en (C₀-C₄)-N(R21)-R22, -alkylène en (C₀-C₄)-N(R21)-C(O)-R22, -alkylène en (C₁-C₆)-O-alkyle en (C₁-C₆), -alkylène en (C₀-C₆)-O-alkylène en (C₁-C₆)-O-alkyle en (C₁-C₆) ou alkylène en (C₀-C₆)-O-alkylène en (C₁-C₄)-cycloalkyle en (C₃-C₆),
alkyle, alkylène et cycloalkyle étant chacun non substitués ou substitués une, deux ou trois fois indépendamment les uns des autres par -alkyle en (C₁-C₄), -cycloalkyle en (C₃-C₆), OH, -O-alkyle en (C₁-C₆) ou -O-cycloalkyle en (C₃-C₆), ou
les atomes d'hydrogène dans alkyle, alkylène ou cycloalkyle pouvant être remplacés en totalité ou en partie par fluor,
à condition qu'au moins un des radicaux R4, R5, R6, R7 et R8 ne représente pas l'hydrogène,
R9 représente un atome d'hydrogène ou -alkyle en (C₁-C₆), les atomes d'hydrogène dans l'alkyle pour être remplacés en totalité ou en partie par fluor,
R21 et R22 représentent indépendamment l'un de l'autre un atome d'hydrogène, -alkyle en (C₁-C₆) ou cycloalkyle en (C₃-C₆),
les atomes d'hydrogène dans l'alkyle ou le cycloalkyle pouvant être remplacés en totalité ou en partie par fluor, ou
R21 et R22 représentent dans les fragments « N(R21)-R22 » et « N(R21)-C(O)-R22 » un cycle choisi dans le groupe constitué par pyrrolidinyle, pipéridinyle, pipérazinyle, morpholinyle, thiomorpholinyle, pyrrolidine-2,5-dionyle, pipéridine-2,6-dionyle, pipérazine-2,6-dionyle, morpholine-3,5-dionyle, pyrrolidin-2-onyle, pipéridin-2-onyle, pipérazin-2-onyle et morpholin-3-onyle, le cycle étant non substitué ou substitué une ou deux fois indépendamment l'une de l'autre par -alkyle en (C₁-C₄), cycloalkyle en (C₃-C₆), OH, -O-alkyle en (C₁-C₆) ou -O-cycloalkyle en (C₃-C₆), les atomes d'hydrogène dans l'alkyle ou le cycloalkyle pouvant être remplacés en totalité ou en partie par fluor.

2. Composé de formule I selon la revendication 1, dans lequel
Q1, Q2 et Q3 sont identiques et représentent chacun un atome d'hydrogène,
R1, R2 et R3 sont identiques ou différents, et représentent indépendamment les uns des autres un atome d'hydrogène, -alkyle en (C₁-C₄), -O-CH₂-CF₃ ou -O-alkyle en (C₁-C₆), à condition qu'au moins un radical parmi R1, R2 et R3 ne représente pas un atome d'hydrogène,
R4 et R8 représentent chacun un atome d'hydrogène, R5, R6 et R7 sont identiques ou différents, et représentent indépendamment les uns des autres un atome d'hydrogène, -alkyle en (C₁-C₆), -O-alkyle en (C₁-C₄), -SF₅ ou N(R21)-R22, à condition qu'au moins un des radicaux R5, R6 ou R7 ne représente pas un atome d'hydrogène, et R9 représente un atome d'hydrogène,
R21 et R22 représentent indépendamment l'un de l'autre un atome d'hydrogène ou -alkyle en (C₁-C₄), ou
R21 et R22 représentent dans le fragment « N(R21)-R22 » un cycle choisi dans le groupe constitué par pyrrolidinyle, pipéridinyle, pipérazinyle, imidazolyle, morpholinyle, thiomorpholinyle, pyrrolidine-2,5-dionyle, pipéridine-2,6-dionyle, pipérazine-2,6-dionyle, morpholine-3,5-dionyle, pyrrolidin-2-onyle, pipéridin-2-onyle, pipérazin-2-onyle et morpholin-3-onyle.

3. Composé de formule I selon la revendication 1, dans lequel le composé de formule I est choisi dans le groupe constitué par la 1-(3-tert-butyl-4-méthoxy-5-morpholin-4-yl-phényl)-2-(7-imino-2,3-diméthoxy-imidazo[1,5-b]pyridazin-6-yl)-éthanone, la 1-(3-tert-butyl-4-méthoxy-5-morpholin-4-yl-phényl)-2-(2,3-diéthoxy-7-imino-imidazo[1,5-b]pyridazin-6-yl)-éthanone, le N-{3-[2-(2,3-diéthoxy-7-imino-imidazo[1,5-b]pyridazin-6-yl)-acétyl]-5-pentafluorosulfanyl-phényl}-acétamide, la 1-(3-tert-butyl-4-méthoxy-5-morpholin-4-yl-phényl)-2-[7-imino-2-méthoxy-3-(2,2,2-trifluoro-éthoxy)-imidazo[1,5-b]pyridazin-6-yl]-éthanone, la 2-(2,3-diéthoxy-7-imino-imidazo[1,5-b]pyridazin-6-yl)-1-(5-méthylamino-3-pentafluorosulfanyl-phényl)-éthanone ou la 2-(2,3-diéthoxy-7-imino-imidazo[1,5-b]pyridazin-6-yl)-1-[3-méthylamino-5-(pentafluorosulfanyl)-phényl]-éthanone.

4. Médicament, **caractérisé par** une teneur efficace en au moins un composé de formule I selon une ou plusieurs des revendications 1 à 3, conjointement avec un véhicule pharmaceutiquement approprié et physiologiquement compatible, un additif et/ou d'autres agents actifs et adjuvants.

5. Utilisation du composé de formule I selon une ou plusieurs des revendications 1 à 3 pour la fabrication d'un médicament pour la prophylaxie, la prévention secondaire et le traitement de toutes les maladies qui accompagnent les thromboses, les embolies, l'hypercoagulabilité, les modifications fibrotiques ou les maladies inflammatoires.

6. Utilisation selon la revendication 5, **caractérisée en ce qu'**il s'agit d'un infarctus du myocarde, d'une angine de poitrine et d'autres formes du syndrome coronarien aigu, d'un accident vasculaire cérébral, des maladies vasculaires périphériques, de la thrombose veineuse profonde, de l'embolie pulmonaire, des évènements emboliques ou thrombotiques causés par des arythmies cardiaques, des évènements cardiovasculaires tels que la resténose après une revascularisation et une angioplastie et des interventions semblables telles que les implantations de stents et les opérations de pontage, ou de la réduction du risque de thrombose après des interventions chirurgicales telles que des opérations du genou et de la hanche, ou d'autres interventions qui conduisent à un contact du sang avec des surfaces étrangères, tel que chez les patients sous dialyse et les patients portant une sonde à demeure, ou de la coagulation intravasculaire disséminée, de la sepsie et d'autres évènements intravasculaires qui sont accompagnés d'une inflammation, de l'athérosclérose, du diabète et du syndrome métabolique et leurs conséquences, de la croissance de tumeurs et de la métastase de tumeurs, des maladies articulaires inflammatoires et dégénératives telles que la polyarthrite rhumatoïde et l'arthrose, des troubles du système hémostatique tels que les dépôts de fibrine, les modifications fibrotiques des poumons telles que la maladie pulmonaire obstructive chronique, le syndrome de détresse respiratoire de l'adulte ou les dépôts de fibrine de l'oeil après des opérations de l'oeil, ou de l'inhibition et/ou du traitement de cicatrices.

7. Procédé de fabrication du composé de formule I selon une ou plusieurs des revendications 1 à 3, **caractérisé en ce que**
a) un composé de formule II 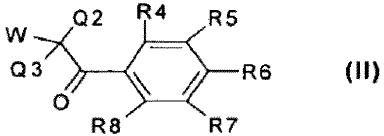 dans laquelle R4, R5, R6, R7, R8, Q2 et Q3 sont tels que définis dans la formule I et W représente chlorure, bromure, mésylate ou tosylate,
est mis en réaction avec un composé de formule III 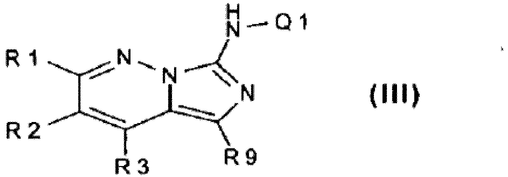 dans laquelle R1, R2, R3, R9 et Q1 sont tels que définis dans la formule I, avec ou sans ajout de base dans un solvant pour former un composé de formule I, ou
b) un composé de formule VII 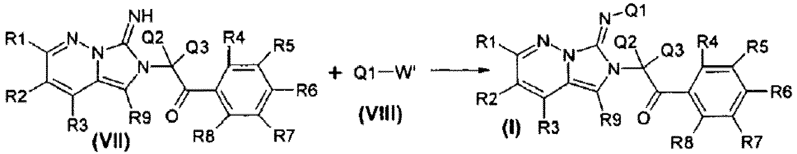 dans laquelle R1, R2, R3, R4, R5, R6, R7, R8, R9, Q1, Q2 et Q3 sont tels que définis dans la formule I, est mis en réaction avec un composé Q1-W', W' signifiant chlorure, bromure, tosylate, mésylate, méthylsulfate ou un bon groupe partant analogue, avec ou sans ajout de base pour former un composé de formule I, ou
c) un composé de formule XXVI 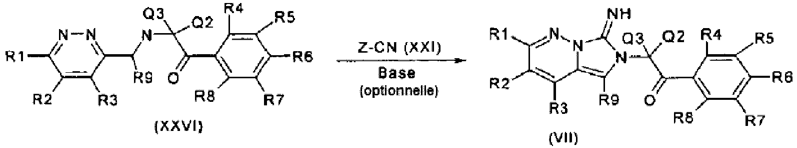 dans laquelle R1, R2, R3, R4, R5, R6, R7, R8, R9, Q1, Q2 et Q3 sont tels que définis dans la formule I, est mis en réaction avec un composé Z-CN, Z signifiant un bon groupe partant tel que tosylate, chlorure ou bromure, avec ou sans ajout de base pour former un composé de formule VII, ou
d) un composé de formule XXVII 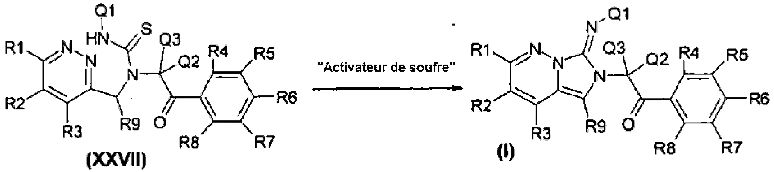 dans laquelle R1, R2, R3, R4, R5, R6, R7, R8, R9, Q1, Q2 et Q3 sont tels que définis dans la formule I, est mis en réaction avec un activateur de soufre pour former un composé de formule I, ou
e) le composé de formule I fabriqué par les procédés a) à d) est isolé sous forme libre ou libéré à partir de sels physiologiquement incompatibles ou, dans le cas de la présence de groupes acides ou basiques, transformé en sels physiologiquement compatibles, ou
f) un composé de formule I fabriqué par les procédés a) à d) ou un précurseur approprié de formule I, qui se présente sous des formes énantiomères ou diastéréomères en raison de sa structure chimique, est séparé en les énantiomères ou diastéréomères purs par formation de sels avec des acides ou des bases énantiomériquement purs, chromatographie sur des phases stationnaires chirales ou dérivation avec des composés énantiomériquement purs chiraux tels que des acides aminés, séparation des diastéréomères ainsi obtenus et clivage des groupes auxiliaires chiraux.
